# EUROPEAN PATENT APPLICATION

(11) **EP 4 748 944 A1**
(43) Date of publication of application: **27.05.2026**
(21) Application number: 24842864.1
(22) Date of filing: 14.06.2024
(51) Int. Cl.: C12Q 1/6848, C12N 15/12

(54) **METHOD FOR PRODUCING CIRCULAR DNA**

(30) Priority: 19.07.2023 JP 2023117904
(71) Applicant: Moderna Enzymatics Co., Ltd., Tokyo 136-0082 (JP)
(72) Inventor: NAKANO, Masahiro, Tokyo 136-0082 (JP); SUZUKI, Shota, Tokyo 136-0082 (JP); SUETSUGU, Masayuki, Tokyo 171-8501 (JP)
(74) Representative: Hoffmann Eitle
(86) International application number: PCT/JP2024/021729
(87) International publication number: WO 2025/018068

(57) **Abstract**

The present invention provides an efficient method for producing circular DNA. This method for producing circular DNA includes: (1) preparing circular DNA that will serve as a template, said circular DNA including (i) a replication initiation sequence capable of binding to an enzyme that has a DnaA activity, and (ii) a gyrase binding sequence, and said circular DNA not including a DnaA box cluster at a different position from the replication initiation sequence; (2) forming a reaction mixture of the circular DNA prepared in (1) and a reaction liquid including a first enzyme group that catalyzes replication of the circular DNA, a second enzyme group that synthesizes two sister circular DNAs forming a catenane, and a third enzyme group that catalyzes a separation reaction of the two sister circular DNAs; and (3) incubating the reaction mixture formed in (2) at a temperature of 80°C or lower, thereby obtaining a reaction product that includes the circular DNA, which has been amplified.

## Description

### Technical Field

The present invention relates to an efficient method for producing circular DNA in a cell-free system and circular DNA used in such a method.

This application claims priority based on Japanese Patent Application No. 2023-117904, filed on July 19, 2023, the contents of which are incorporated herein by reference.

### Background Art

With the advancement of biotechnology, technological developments for in-vitro nucleic acid amplification are progressing. In particular, the replication cycle reaction (RCR) method is available as a technique for amplifying circular DNA in a cell-free system (Patent Documents 1 to 3 and Non-Patent Documents 1 and 2).

### Citation List

### Patent Documents

Patent Document 1: WO 2016/080424 A
Patent Document 2: WO 2017/199991 A
Patent Document 3: WO 2018/159669 A

### Non-Patent Documents

Non-Patent Document 1: Su'etsugu et al., Nucleic Acids Research, 2017, vol. 45(20), p. 11525-11534.
Non-Patent Document 2: Hasebe, et al., Life, 2018, vol. 8(4), p. 43.

### Summary of Invention

### Technical Problem

Circular DNA, particularly circular double-stranded DNA, is used in various situations, including mRNA synthesis in which circular DNA is used as a template plasmid, and demand therefor is increasing. Under this circumstance, the primary object of the present invention is to provide an efficient method for producing circular DNA.

### Solution to Problem

As a result of intensive research, the present inventors discovered that the amplification efficiency of the conventional RCR method is further increased when the circular DNA to be amplified has a specific sequence, thus completing the present invention.

That is, the present invention relates to the following.
[1] A method for producing circular DNA, the method including: (1) preparing circular DNA serving as a template, the circular DNA including (i) a replication initiation sequence capable of binding to an enzyme that has a DnaA activity, and (ii) a gyrase binding sequence, and
   the circular DNA not including a DnaA box cluster at a different position from the replication initiation sequence;
   (2) forming a reaction mixture of the circular DNA prepared in (1) and a reaction liquid including
   a first enzyme group that catalyzes replication of the circular DNA,
   a second enzyme group that synthesizes two sister circular DNAs forming a catenane, and
   a third enzyme group that catalyzes a separation reaction of the two sister circular DNAs; and
   (3) incubating the reaction mixture formed in (2) at a temperature of 80°C or lower, thereby obtaining a reaction product that includes the circular DNA, which has been amplified.
[2] The method according to [1], in which the replication initiation sequence in (i) has a mutant double-strand cleavage region with a higher AT content than the double-strand cleavage region of a wild-type replication initiation sequence, the double-strand cleavage region having L, M, and R elements.
[3] The method according to [1] or [2], in which the gyrase binding sequence in (ii) is a sequence derived from bacteriophage Mu.
[4] A method for producing circular DNA, the method including: (1) preparing circular DNA serving as a template, the circular DNA including (i) a replication initiation sequence which is capable of binding to an enzyme having a DnaA activity, and
   has a mutant double-strand cleavage region with a higher AT content than the double cleavage region of a wild-type replication initiation sequence, the double-strand cleavage region having L, M, and R elements;
   (2) forming a reaction mixture of the circular DNA prepared in (1) and a reaction liquid including
   a first enzyme group that catalyzes replication of the circular DNA,
   a second enzyme group that synthesizes two sister circular DNAs forming a catenane, and
   a third enzyme group that catalyzes a separation reaction of the two sister circular DNAs; and
   (3) incubating the reaction mixture formed in (2) at a temperature of 80°C or lower, thereby obtaining a reaction product that includes the circular DNA, which has been amplified.
[5] The method according to [4], following (3), further including:
   (4) diluting the reaction product two-fold or more with a solution that does not contain the first to third enzyme groups, and maintaining the diluted product obtained after the dilution under conditions in which the second enzyme group and the third enzyme group act.
[6] The method according to any one of [2] to [5], in which the mutant double-strand cleavage region is
   a mutant double-strand cleavage region in which, among the L, M, and R elements constituting the double-strand cleavage region, the M and/or R elements have a higher AT content than the wild-type M and/or R elements,
   a mutant double-strand cleavage region in which the M element constituting the double-strand cleavage region has repetitive AT sequences, and/or
   a mutant double-strand cleavage region in which the R element constituting the double-strand cleavage region has four or more consecutive KAK (K is T or G) motif sequences.
[7] The method according to any one of [1] to [6], in which the circular DNA further includes a ter sequence inserted outward with respect to the replication initiation sequence,
   the ter sequence is not adjacent to the replication initiation sequence, and
   the reaction liquid in (2) further includes a protein having an activity of inhibiting replication by binding to the ter sequence.
[8] The method according to any one of [1] to [7], in which the circular DNA further includes a ter sequence inserted outward with respect to the replication initiation sequence capable of binding to an enzyme having a DnaA activity,
   the ter sequence is a ter sequence with the introduction of a mutation causing a substitution, deletion, or insertion of one or more bases in the wild-type ter sequence, and
   the reaction liquid in (2) further includes a protein having an activity of inhibiting replication by binding to the ter sequence.
[9] The method according to any one of [1] to [8], in which the incubation in (3) includes isothermal incubation.
[10] The method according to any one of [1] to [8], in which the incubation in (3) includes incubation under temperature cycles repeating incubation at two temperatures of 65°C or lower.
[11] The method according to any one of [1] to [10], in which the first enzyme group includes an enzyme having a DnaA activity, an SSB, an enzyme having a DnaB-type helicase activity, an enzyme having a DNA helicase loader activity, an enzyme having a DNA primase activity, an enzyme having a DNA clamp activity, and an enzyme or enzyme group having a DNA polymerase III activity,
   the second enzyme group includes an enzyme having a DNA polymerase I activity and an enzyme having a DNA ligase activity, and
   the third enzyme group includes an enzyme having a topoisomerase III activity or an enzyme having a topoisomerase IV activity.
[12] The method according to any one of [1] to [11], in which the reaction liquid further includes a linear DNA-specific exonuclease.
[13] The method according to any one of [1] to [12], in which the reaction liquid further includes a single-stranded DNA-specific exonuclease.
[14] Circular DNA, including: (i) a replication initiation sequence capable of binding to an enzyme that has a DnaA activity; and (ii) a gyrase binding sequence,
   in which the circular DNA does not include a DnaA box cluster at a different position from the replication initiation sequence.
[15] Circular DNA, including: (i) a replication initiation sequence capable of binding to an enzyme that has a DnaA activity; (ii) a gyrase binding sequence; and (iii) a plasmid replication origin,
   in which the circular DNA does not include a DnaA box cluster at a different position from the replication initiation sequence.

### Advantageous Effects of Invention

According to the present invention, circular DNA can be efficiently produced. It is also possible to provide circular DNA which can be efficiently produced by the RCR method.

### Brief Description of Drawings

[Fig. 1] Fig. 1 is a view of the gel electrophoresis in Example 5.
[Fig. 2] Fig. 2 is a view of the gel electrophoresis in Example 6.
[Fig. 3] Fig. 3 is a view of the gel electrophoresis in Example 7.
[Fig. 4] Fig. 4 is a view of the gel electrophoresis in Example 8.
[Fig. 5] Fig. 5 is a view of the gel electrophoresis in Example 9-1.
[Fig. 6] Fig. 6 is a view of the gel electrophoresis in Example 9-2.
[Fig. 7] Fig. 7 is a view of the gel electrophoresis in Example 10.
[Fig. 8] Fig. 8 is a view of the gel electrophoresis in Example 11.
[Fig. 9] Fig. 9 is a graph showing the change in DNA concentration with reaction time in Example 11.
[Fig. 10] Fig. 10 is a view of the gel electrophoresis in Example 12.
[Fig. 11] Fig. 11 is a view of the gel electrophoresis in Example 13.
[Fig. 12] Fig. 12A shows a schematic diagram of the circular DNA amplification cycle by the RCR method. Fig. 12B shows a schematic diagram of a process of initiating replication from oriC.
[Fig. 13] Fig. 13 shows the results of Example 14-2. Fig. 13A shows the sequences of the wild type and two types of oriC cassette mutants. Fig. 13B shows the results of amplifying DNAs with different GC contents using the three types of oriC cassettes. Fig. 13C shows the results of real-time RCR performed using the three types of oriC cassettes.
[Fig. 14] Fig. 14 shows the results of Example 14-3. Fig. 14A shows the sequences of the wild type and two types of oriC cassette mutants. Fig. 14B is a schematic diagram showing the positions of mutations in four types of oriC cassette mutants. Fig. 14C shows the results of real-time RCR performed using five types of oriC cassettes. Fig. 14D shows the results of amplifying DNAs with different GC contents using four types of oriC cassettes.
[Fig. 15] Fig. 15 shows the results of Example 14-4. Fig. 15A shows the sequences of the wild type and two types of oriC cassette mutants. Fig. 15B is a schematic diagram showing the positions of mutations in three types of oriC cassette mutants. Fig. 15C shows the results of real-time RCR performed using four types of oriC cassettes. Fig. 15D shows the sequences of the wild type and four types of oriC cassette mutants.
[Fig. 16] Fig. 16 shows the results of Example 14-5. Fig. 16A shows the results of performing DNA amplification by the RCR method at different temperatures using seven types of oriC cassettes. Fig. 16B shows the results of competitive RCR amplification performed using five types of oriC cassettes.
[Fig. 17] Fig. 17 is a view of the gel electrophoresis in Example 15.
[Fig. 18] Fig. 18 is a graph showing the change in DNA concentration with reaction time in Example 15.
[Fig. 19] Fig. 19 is a view of the gel electrophoresis in Example 16.
[Fig. 20] Fig. 20 is a view of the gel electrophoresis in Example 17.
[Fig. 21] Fig. 21 is a graph showing the change in DNA concentration with reaction time in Example 18.
[Fig. 22] Fig. 22 is a graph showing the change in DNA concentration with reaction time in Example 19.

### Description of Embodiments

Hereinafter, an embodiment of the present invention (also referred to as the present embodiment) will be described, but the present invention is not limited thereto. Unless otherwise particularly defined herein, the scientific and technical terms used in connection with the present invention shall have the meaning generally understood by those skilled in the art.

### <Circular DNA>

In the present embodiment, circular DNA used as the template is preferably double stranded. The circular DNA used as a template is not particularly limited as long as it includes a replication initiation sequence capable of binding to an enzyme that has a DnaA activity and has the characteristics described below. Examples of the circular DNA used as a template include circular DNA obtained by ligating natural circular DNA such as circular chromosomes of microorganisms cut by an enzymatic treatment or the like to another DNA fragment and circularizing the ligated product, and all artificially synthesized circular DNAs.

As the replication initiation sequence capable of binding to an enzyme that has a DnaA activity, for example, a known replication initiation sequence present in bacteria such as Escherichia coli and Bacillus subtilis can be obtained from a public database such as NCBI. The replication initiation sequence can also be obtained by cloning a DNA fragment capable of binding to an enzyme that has a DnaA activity and analyzing the base sequence thereof. As the replication initiation sequence used in the present invention, a modified sequence capable of binding to an enzyme that has a DnaA activity, which is a sequence obtained by introducing a mutation that causes a substitution, deletion, or insertion of one or two or more bases in a known replication initiation sequence, can also be used. The replication initiation sequence used in the present invention is preferably oriC and a modified sequence thereof, more preferably oriC derived from Escherichia coli and a modified sequence thereof. The circular DNA used as a template in the present embodiment may be circular DNA which originally includes a replication initiation sequence, or may be circular DNA obtained by introducing a replication initiation sequence into circular DNA which does not originally contain a replication initiation sequence.

In one aspect, the replication initiation sequence capable of binding to an enzyme that has a DnaA activity is oriC derived from Escherichia coli and a modified sequence thereof, the sequence having a length of 265 bp or more. In one aspect, the replication initiation sequence capable of binding to an enzyme that has a DnaA activity is a sequence having SEQ ID NO: 4 or a modified sequence of SEQ ID NO: 4, the sequence having no mutations in the 12 or 13 bases at either end of SEQ ID NO: 4. In one aspect, the replication initiation sequence capable of binding to an enzyme that has a DnaA activity is a modified sequence of SEQ ID NO: 4, which is a sequence having SEQ ID NO: 29 (5' end) and/or SEQ ID NO: 30 (3' end) that are underlined below, or a sequence having a deletion, substitution, or addition of one or a plurality of bases in SEQ ID NO: 29 or 30.

**[Table 1]**

| |
|---|
| Example of replication initiation sequence capable of binding to enzyme that has DnaA activity (oriC sequence derived from Escherichia coli chromosome: SEQ ID NO: 4) |
| GGATCCTGGGTATTAAAAAGAAGATCTATTTATTTAGAGATCTGTTCTATTGTGATCTCTTATTAGGATCGCACTGCCCTG TGGATAACAAGGATCCGGCTTTTAAGATCAACAACCTGGAAAGGATCATTAACTGTGAATGATCGGTGATCCTGGACCGTA TAAGCTGGGATCAGAATGAGGGGTTATACACAACTCAAAAACTGAACAACAGTTGTTCTTTGGATAACTACCGGTTGATCC AAGCTTCCTGACAGAGTTATCCACAGTAGATCGCACGATCTGT |

In one aspect, the replication initiation sequence capable of binding to an enzyme that has a DnaA activity is a modified sequence of oriC in which one or a plurality of the 11 DnaA boxes present in oriC have been deleted. The DnaA boxes in oriC are referred to as R1, R5, τ2, I1, I2, R2, C3, C2, I3, C1, and R4 from the side close to DUE. In one aspect, the modified sequence of oriC is a sequence in which, among the 11 DnaA boxes, 1 to 6 selected from R2, C3, C2, I3, C1, and R4, for example, 1 to 5 selected from C3, C2, I3, C1, and R4, for example, C3, C2, I3, C1, and R4 have been deleted.

In one aspect, the circular DNA used in the method of the present embodiment includes (i) a replication initiation sequence capable of binding to an enzyme that has a DnaA activity and (ii) a gyrase binding sequence (strong gyrase binding sequence, SGS). SGS is a binding sequence to which DNA gyrase binds, thereby introducing negative supercoiling into the DNA. Examples of the SGS include a sequence including RNNNRNR[T/G]GRYC[G/T]YNYN[G/T]NY (R = A or G, Y = C or T, N = A, G, C, or T) (SEQ ID NO: 31), which is the consensus sequence thereof, or a sequence complementary thereto (Lockshon and Morris, Journal of Molecular Biology, 1985, vol. 181, p. 63-74.). The SGS may be a sequence derived from any biological species as long as it has the consensus sequence. For example, SGS derived from a phage or SGS derived from a plasmid can be used. In addition, the SGS may have a sequence before or after the consensus sequence, or may be a modified sequence of SGS derived from any biological species. The modified sequence refers to a base sequence into which a mutation that causes a substitution, deletion, or insertion of one or two or more bases in the base sequence before the modification has been introduced, while maintaining the function of increasing the efficiency of amplifying the circular DNA including the modified sequence by the RCR method. In one aspect, the SGS is preferably SGS derived from a bacteriophage, more preferably SGS derived from the bacteriophage Mu (Mu-SGS) (Folarin et al., Bioengineering, 2019, vol. 6(2):54.), more preferably the sequence of SEQ ID NO: 9 or a sequence including the sequence, from the viewpoint of obtaining a sufficient effect of increasing the amplification efficiency of the circular DNA in the replication initiation sequence.

The SGS may be located at any position in the circular DNA, and the distance of the SGS from the replication initiation sequence is not particularly limited. In one aspect, the SGS is adjacent or in proximity to the replication initiation sequence and is located within 30 bases, 20 bases, 15 bases, 13 bases, or 10 bases from the end of the replication initiation sequence. In one aspect, the SGS is adjacent or in proximity to the end of the replication initiation sequence on the double-strand cleavage region (DUE) side and is located within 30 bases, 20 bases, 15 bases, 13 bases, or 10 bases from the end of the replication initiation sequence on the DUE side.

In one aspect, the circular DNA used in the method of the present embodiment includes (i) a replication initiation sequence capable of binding to an enzyme that has a DnaA activity and (ii) a gyrase binding sequence, and does not include a DnaA box cluster at a different position from the replication initiation sequence. In one aspect, the circular DNA used in the method of the present embodiment includes (i) a replication initiation sequence capable of binding to an enzyme that has a DnaA activity, (ii) a gyrase binding sequence, and (iii) a plasmid replication origin, and does not include a DnaA box cluster at a different position from the replication initiation sequence.

The circular DNA having such a configuration can be efficiently amplified when amplified by the RCR method described later. More particularly, the circular DNA having such a configuration is useful in terms of one or more of the amplification at a higher yield, amplification at a higher amplification rate, amplification with a smaller amount of enzyme, and accurate amplification from a lower concentration (for example, one molecule) of circular DNA.

When the circular DNA according to the present embodiment has a plasmid replication origin, examples thereof include well-known plasmid replication origins such as the p15A-type, F-type, ColE1-type, psC101-type, P1-type, R1-type, R6Kγ-type, λ-type, φB2-type, φB0-type, RK2-type, and P4-type replication origins, and the p15A-type and F-type replication origins are particularly preferable. Examples of the p15A-type replication origin include p15A, which is the replication origin of a plasmid such as pACYC, or a modified sequence thereof. Examples of the F-type replication origin include fori, which is the replication origin of a plasmid such as pBeloBAC, or a modified sequence thereof. As the modified sequence, it is possible to use a base sequence into which a mutation that causes a substitution, deletion, or insertion of one or two or more bases in the base sequence before the modification has been introduced, while maintaining the function as a plasmid replication origin. In one aspect, when the circular DNA according to the present embodiment has a plasmid replication origin, it is not of the ColE1 type. Examples of the ColE1-type replication origin include pMB1, which is the replication origin of plasmids such as pUC, pGEM, pTZ, and pBR322, and ColE1, which is the replication origin of plasmids such as pBluescript.

In one aspect of the present embodiment, the circular DNA serving as a template does not include a DnaA box cluster at a different position from the replication initiation sequence. The DnaA box cluster is a region in which two or more DnaA boxes or analogous sequences thereof, either forward or reverse, are present in proximity to each other within 50 bases. The consensus sequence of the DnaA box is TT[A/T]TNCACA (DnaAB consensus sequence 1), and the reverse sequence thereof is TGTGNA[A/T]AA (DnaAB consensus sequence 2), and an analogous sequence thereof is a sequence in which one base or a plurality of bases, for example, 1, 2, 3, 4, or 5 bases, preferably 1 to 3 bases, more preferably 1 or 2 bases in the DnaAB consensus sequence 1 or the DnaAB consensus sequence 2 have been deleted, substituted, or added. The p15A which is the p15A-type replication origin and the fori which is the F-type replication origin do not include the DnaA box cluster, and the pUC which is the ColE1-type replication origin includes the DnaA box cluster.

In one aspect, the circular DNA of the present embodiment is circular DNA that does not have two or more, for example, three, of the DnaAB consensus sequence 1, the DnaAB consensus sequence 2, and a sequence in which one to three, preferably one or two, bases have been deleted. substituted, or added in these sequences within a region of 100 bases, 90 bases, 80 bases, 70 bases, or 60 bases, preferably, within a region of 50 bases (in the case of two, for example, within a region of 50 bases, 40 bases, or 30 bases), the region being at a different position from the replication initiation sequence. In one aspect, the circular DNA of the present embodiment is not circular DNA that includes the DnaAB consensus sequence 1 or an analogous sequence thereof, the DnaAB consensus sequence 2 or an analogous sequence thereof, and the DnaAB consensus sequence 2 or an analogous sequence thereof within 50 bases in this order. Here, the analogous sequence is a sequence in which one or two bases have been deleted, substituted, or added in the original sequence. In one aspect, the circular DNA of the present embodiment is not circular DNA that includes the DnaAB consensus sequence 1 or an analogous sequence thereof, an analogous sequence of the DnaAB consensus sequence 2, and an analogous sequence of the DnaAB consensus sequence 2 within 50 bases in this order. Here, the analogous sequence is a sequence in which one or two bases have been deleted, substituted, or added in the original sequence.

The replication initiation sequence (for example, oriC) capable of binding to an enzyme that has a DnaA activity includes DUE and a DnaA assembly region (DOR). When ATP-bound DnaA binds to the replication initiation sequence, the DnaA-oriC complex changes its structure, and the double-stranded DNA is cleaved within the DUE region, initiating DNA replication. In one embodiment, the circular DNA used in the method of the present embodiment includes (i) a replication initiation sequence capable of binding to an enzyme that has a DnaA activity and (ii) a gyrase binding sequence, and (i) the replication initiation sequence capable of binding to an enzyme that has a DnaA activity has one or more of the following features (F1) to (F5).
(F1) Mutant DUE with a higher AT content than the wild-type DUE is present.
(F2) The DUE has repetitive AT sequences in the M element.
(F3) The DUE has four or more consecutive KAK (K is T or G) motif sequences in the R element.
(F4) The R5M sequence in the DOR region has a sequence identical to the consensus sequence of DnaA (TGTGNAWAA).
(F5) Compared to the wild-type DUE, the DUE region has an increased number of TT[A/G]T(T) sequence motifs (for example, TTATT).

The circular DNA of the present embodiment has a mutant DUE with a higher AT content than the wild-type DUE.

A higher AT content in the mutant sequence as used herein refers to having 1 or more or 2 or more, preferably 3 or more, 4 or more, 5 or more, 6 or more, 7 or more, 8 or more, or 9 or more mutations compared to the wild-type sequence before the mutation. The mutation is a deletion of C or G or a substitution of C or G by A or T, the latter being more preferable.

The DUE has three elements of 13 bases with a high AT content which are referred to as the L, M, and R elements, respectively (Katayama et al., Frontiers in Microbiology (2017) Vol. 8:2496.). In one aspect, the circular DNA of the present embodiment has a mutant DUE with a higher AT content than the wild-type DUE. Preferably, the AT contents in at least one, preferably at least two elements in the mutant DUE are higher than those in the wild-type DUE. More preferably, the AT content in the M or R element, even more preferably, the AT contents in the M and R elements are higher in the mutant DUE of the circular DNA of the present embodiment than those in the wild-type DUE. The mutant DUE with a higher AT content than the wild-type DUE is, for example, a sequence having an increased number of AT motifs (a portion where A and T are consecutive) compared to the wild-type DUE.

The sequences of the wild-type DUE and the L, M, and R elements of the wild-type DUE are shown below. Each DUE is a 13-mer having the sequence identical to the consensus sequence (GATCTnTTnTTTT: SEQ ID NO: 110) (Bramhill, D., & Kornberg, A. (1988) Cell, 52(5), 743-755.). In one aspect, the circular DNA of the present embodiment has one or more or two or more, preferably three or more, four or more, five or more, six or more, seven or more, eight or more, or nine or more mutations in one or more sequences thereof, for example, SEQ ID NO: 111. In one aspect, the circular DNA of the present embodiment has one or more, preferably two or more, three or more, or four or more mutations in SEQ ID NO: 113 or SEQ ID NO: 114, or SEQ ID NO: 113 and SEQ ID NO: 114, respectively. The mutations are deletions of C or G or substitutions of C or G by A or T, preferably substitutions of C or G by A or T.

**[Table 2]**

| | Sequence (underlined portions represent each element) | SEQ ID NO |
|---|---|---|
| wt_DUE | GATCTATTTATTTAGAGATCTGTTCTATTGTGATCTCTTATTAG | 111 |
| wt_L element | GATCTATTTATTT | 112 |
| wt_M element | GATCTGTTCTATT | 113 |
| wt_R element | GATCTCTTATTAG | 114 |

In one aspect, the circular DNA of the present embodiment preferably has repetitive AT sequences, for example, an (AT)n sequence, in the M element of the DUE, as compared to the wild-type DUE. Here, n is an integer of 2 or more, preferably an integer of 3 or more, 4 or more, or 5 or more. Although the upper limit of n is not particularly limited, n is an integer of, for example, 30 or less, preferably 25 or less, 20 or less, or 15 or less, and in one aspect, n is an integer of, for example, 12 or less, 10 or less, or 8 or less. For example, n is an integer of 3 to 10, an integer of 3 to 8, an integer of 5 to 8, an integer of 5 to 7, or 7. The repetitive AT sequences in the M element may include a sequence other than the M element, as long as the M element sequence is included in the repetitive sequences. In one aspect, the circular DNA of the present embodiment may have the repetitive AT sequences in the 5' sequence of the M element. The repetitive AT sequences in the M element may include a plurality of non-consecutive repetitive AT sequences, as long as the AT sequences are repeated and the M element sequence is included in the repetitive sequences. For example, certain circular DNA may have an (AT)_{n'} sequence and an (AT)_{n"} sequence in the M element of the DUE, in which case the M element of the DUE has an (AT)ₙ sequence (here, n = n' + n"). In a preferred aspect, for example, the M element of the DUE (including a surrounding sequence thereof) in the circular DNA of the present embodiment has a sequence selected from GATATGTTCTATT (SEQ ID NO: 50), TATATATTCTATT (SEQ ID NO: 51), TATATATTATATT (SEQ ID NO: 32), TTATTATATTAAC (SEQ ID NO: 52), ATATATATATTCTATT (SEQ ID NO: 53), and ATATATATATTATATT (SEQ ID NO: 54) or a sequence in which one or more bases (preferably one or more bases other than AT) in these sequences have been substituted, deleted, or inserted.

In one aspect, the circular DNA of the present embodiment has mutant DUE that has more consecutive KAK motif sequences (K is T or G; the same applies throughout the present specification unless otherwise noted) in the R element of the DUE compared to the wild-type DUE. Here, the consecutive KAK motif sequences in the R element may include a sequence other than the R element, that is, may include surrounding sequences with the R element in the center, as long as the R element sequence is included in the consecutive sequences. For example, in the wild-type oriC, there are three consecutive KAK motif sequences in the R element of the DUE, but in the circular DNA of the present embodiment, it is preferable that there are 4 or more, 5 or more, 6 more, or 7 or more consecutive KAK motif sequences. The upper limit of the number of KAK motif sequences in the circular DNA of the present embodiment is not particularly limited, but is preferably, for example, 15 or less, 10 or less, or 7 or less. For example, the R element of the DUE can have 5 to 7 consecutive KAK motif sequences. In a preferred aspect, for example, the R element of the DUE (including a surrounding sequence thereof) in the circular DNA of the present embodiment has a sequence selected from GATTATTATTAG (SEQ ID NO: 55), GATTATTATTAGGAT (SEQ ID NO: 56), TATTATTATTAG (SEQ ID NO: 33), and TATTATTATTAGGAT (SEQ ID NO: 57), a sequence in which T has been substituted by G, or G has been substituted by T in the KAK motifs of these sequences, or a sequence in which or one or more bases (preferably one or more bases other than those of the KAK motif sequence) in these sequences have been substituted, deleted, or inserted. Without being bound by theory, it appears that the presence of such KAK motif sequences strengthens the binding of a DnaA protein to the DUE region.

In one aspect, it is preferable that the DUE in the circular DNA of the present embodiment has an (AT)n sequence in the M element, where n is an integer of 3 or more, and the DUE has five or more consecutive KAK motif sequences in the R element.

In one aspect, the circular DNA of the present embodiment has an increased number of TT[A/G]T(T) sequence motifs (for example, TTATT) in the DUE compared to that in the wild-type DUE, and preferably has two or more, for example, three or more, TT[A/G]T(T) sequences (for example, TTATT).

In one embodiment, the DUE sequence in the circular DNA of the present embodiment is preferably a sequence having TATATATTATATT (SEQ ID NO: 32) and TATTATTATTAG (SEQ ID NO:33) or analogous sequences thereof. These analogous sequences are sequences in which one or a plurality of, for example, 1, 2, 3, 4, or 5 bases, preferably 1 to 3 bases, more preferably 1 or 2 bases, particularly preferably one base in SEQ ID NO: 32 or 33 has been deleted, substituted or added.

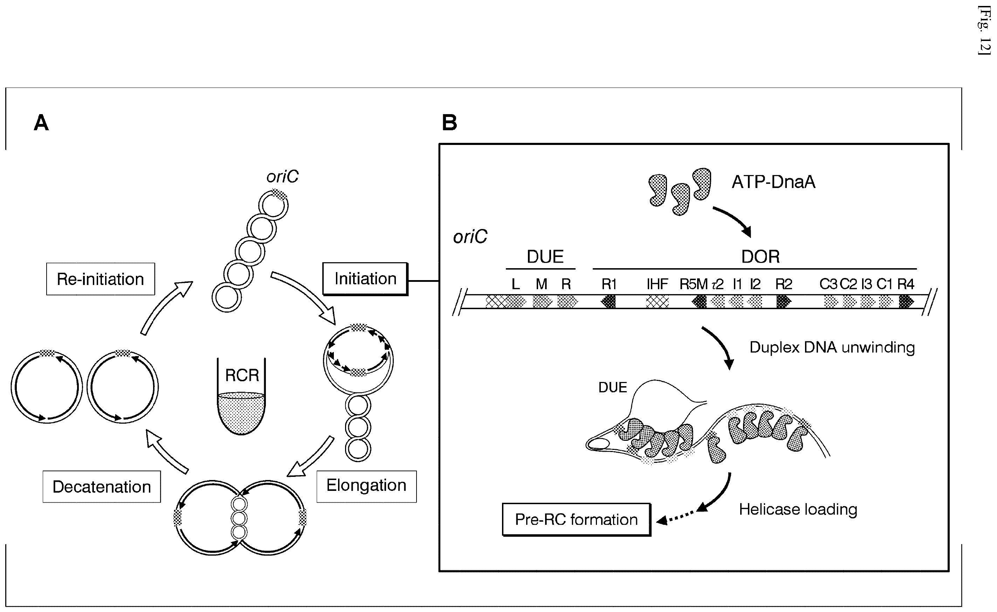

In the DOR, there are 11 DnaA-boxes within the minimal oriC region (Fig. 12B), of which three (R1, R2, and R4) have identical sequences 5'-TGTGnA[T/A]AA-3', whereby the DOR exhibits high affinity for DnaA. Meanwhile, the remaining eight (R5M, τ2, 11, l2, C3, C2, l3, and C1) have 1 to 6 mutations that result in low affinity for DnaA.

In one aspect, the R5M sequence of the DOR in the circular DNA of the present embodiment has a sequence identical to the consensus sequence of DnaA (TGTGNAWAA; N = A, T, G, or C, and W = A or T).

The circular DNA having such a configuration (circular DNA having oriC with such a configuration) can be efficiently amplified when amplified by the RCR method described later. More particularly, the circular DNA having such a configuration is useful in terms of one or more of the amplification at a higher yield, amplification at a higher amplification rate, amplification with a smaller amount of enzyme, and amplification at a lower temperature. Moreover, the circular DNA having such a configuration can be amplified more accurately even when the AT content in the entire circular DNA is high.

In one aspect, the circular DNA of the present embodiment includes (i) a replication initiation sequence capable of binding to an enzyme that has a DnaA activity and (ii) a gyrase binding sequence, the circular DNA not including a DnaA box cluster at a different position from the replication initiation sequence and having an (AT)n sequence in the M element of DUE, where n is an integer of 3 or more, and five or more consecutive KAK motif sequences in the R element of the DUE.

The circular DNA having such a configuration (circular DNA having oriC with such a configuration) can be efficiently amplified when amplified by the RCR method described later. More particularly, the circular DNA having such a configuration is useful in terms of one or more of the amplification at a higher yield, amplification at a higher amplification rate, amplification with a smaller amount of enzyme, and amplification at a lower temperature. Moreover, the circular DNA having such a configuration can be amplified more accurately with less by-products such as concatemers.

The circular DNA of the present embodiment may further have a ter sequence inserted outward with respect to the replication initiation sequence. In one aspect, the ter sequence is inserted outward with respect to the end of the replication initiation sequence far from DUE. In one aspect, the ter sequence is inserted outward with respect to the end of the replication initiation sequence close to the DUE. Thus, when a plasmid including the DNA cassette is amplified in vitro by the RCR method, the generation of a DNA multimer is suppressed, and the amplification product of the plasmid can be efficiently obtained.

The description that the ter sequence is "inserted outward with respect to the replication initiation sequence" means that the ter sequence is inserted in an orientation in which, due to the action of the combination thereof with a protein having an activity of inhibiting replication by binding to the ter sequence, the replication in the direction outward with respect to the replication initiation sequence such as oriC is allowed to occur, while the replication in the direction toward the replication initiation sequence is not allowed to occur, thus being arrested.

In one aspect, the circular DNA of the present embodiment further has a pair of ter sequences each inserted outward with respect to the replication initiation sequence. The description that the ter sequences are present in "a pair each inserted outward with respect to the replication initiation sequence" means that one is inserted at the 5' side of the replication initiation sequence, and the other is inserted at the 3' side of the replication initiation sequence.

Examples of the ter sequence to be inserted at the 5' side of the replication initiation sequence include the sequences set forth in SEQ ID NOs: 34 to 49 described later. Examples of the ter sequence to be inserted at the 3' side of the replication initiation sequence include a sequence including a sequence complementary to the base sequence inserted at the 5' side of the replication initiation sequence.

For example, the Tus-ter system in Escherichia coli and the RTP-ter system in bacteria of the genus Bacillus are known as the replication termination systems that use a protein having an activity of inhibiting replication by binding to the ter sequence on DNA and the ter sequence (Patent Document 3). As the ter sequence, a ter sequence that can be used for these systems or a modified sequence thereof can be used. The modified sequence of the ter sequence refers to a base sequence into which a mutation that causes a substitution, deletion, or insertion of one or two or more bases in the base sequence before the modification has been introduced, while maintaining the function as the ter sequence. When the Tus-ter system is used, the Tus protein is used for the plasmid replication, and when the RTP-ter system is used, the RTP protein is used for the plasmid replication.

The ter sequence in the circular DNA according to the present embodiment may be a wild-type ter sequence or a mutant ter sequence into which a mutation that causes a substitution, deletion, or insertion of one or more bases in the wild-type ter sequence has been introduced. Examples of the wild-type ter sequence that can be used in the Tus-ter system include 5'-GN[A/G][T/A]GTTGTAAC[T/G]A-3' (SEQ ID NO: 34), and a base sequence including 5'-G[T/G]A[T/A]GTTGTAAC[T/G]A-3' (SEQ ID NO: 35), 5'-GTATGTTGTAACTA-3' (SEQ ID NO: 36), 5'-AGTATGTTGTAACTAAAG-3' (SEQ ID NO: 37), 5'-GGATGTTGTAACTA-3' (SEQ ID NO: 38), 5'-GTATGTTGTAACGA-3' (SEQ ID NO: 39), 5'-GGATGTTGTAACTA-3' (SEQ ID NO: 40), 5'-GGAAGTTGTAACGA-3' (SEQ ID NO: 41), or 5'-GTAAGTTGTAACGA-3' (SEQ ID NO: 42) is preferable. As the ter sequence in the circular DNA according to the present embodiment, a mutant ter sequence is preferable. Examples of the mutant ter sequence include a base sequence including a modified sequence in which one or a plurality of bases in the base sequence of SEQ ID NO: 34 have been substituted. As the mutant ter sequence in the circular DNA of the present embodiment, 5'-GN[A/G][T/A]GTTGTACC[T/G]A-3' (SEQ ID NO: 43) and 5'-GTATGTTGTAcCTA-3' (SEQ ID NO: 44) are preferable, and examples thereof include 5'-AGTATGTTGTACCTAAAG-3' (SEQ ID NO: 5) having SEQ ID NO: 44.

Examples of the ter sequence that can be used in the RTP-ter system include a base sequence including 5'-AC[T/A][A/G]ANNNNN[C/T]NATGTACNAAAT-3' (SEQ ID NO: 45). As the ter sequence for use in the RTP-ter system of the DNA cassette according to the present invention, a base sequence including 5'-ACTAATT[A/G]A[A/T]C[T/C]ATGTACTAAAT-3' (SEQ ID NO: 46), 5'-ACTAATT[A/G]A[A/T]C[T/C]ATGTACTAAATTTTCA-3' (SEQ ID NO: 47), 5'-GAACTAATTAAACTATGTACTAAATTTTCA-3' (SEQ ID NO: 48), or 5'-ATACTAATTGATCCATGTACTAAATTTTCA-3' (SEQ ID NO: 49) is preferable.

The location of the ter sequence is not particularly limited as long as the ter sequence is inserted outward with respect to the replication initiation sequence, and the ter sequence may be located between the replication initiation sequence and the SGS or outside the region between the replication initiation sequence and the SGS. In one aspect, it is preferable that the ter sequence is not adjacent to the replication initiation sequence, and when the circular DNA has a pair of ter sequences each located outward with respect to the replication initiation sequence, it is preferable that at least one of the ter sequences is not adjacent to the replication initiation sequence. For example, it is preferable that the distance between the base at 3'-end of at least one ter sequence and the end of the replication initiation sequence (preferably, the end of the replication initiation sequence on the side far from the DUE) is 5 bases long or more, for example, 10 bases long or more. The circular DNA having such a configuration can be efficiently amplified when amplified by the RCR method described later. More particularly, the circular DNA having such a configuration is useful in terms of one or more of the amplification at a higher yield, amplification with a smaller amount of enzyme, and amplification at a higher amplification rate.

In one aspect, the circular DNA of the present embodiment may further have one or a pair of ter sequences inserted inward with respect to the replication initiation sequence. The description that the ter sequence is "inserted inward with respect to the replication initiation sequence" means that the ter sequence is inserted in an orientation in which, due to the action of the combination thereof with a protein having an activity of inhibiting replication by binding to the ter sequence, the replication in the direction outward with respect to the replication initiation sequence such as oriC is not allowed to occur, thus being arrested, while the replication in the direction toward the replication initiation sequence is allowed to occur. In this case, it is preferable that the ter sequence is not adjacent or in proximity to the replication initiation sequence. In one aspect, it is preferable that one or a pair of ter sequences inserted inward with respect to the replication initiation sequence is located 50 bases or more, 75 bases or more, 100 bases or more, 150 bases or more, or 200 bases or more away from the replication initiation sequence.
(a) In one aspect, the circular DNA produced by the method of the present embodiment includes (i) a replication initiation sequence capable of binding to an enzyme that has a DnaA activity and (ii) a gyrase binding sequence.
(b) In one aspect, the circular DNA produced by the method of the present embodiment includes a modified sequence of oriC as a replication initiation sequence capable of binding to an enzyme that has a DnaA activity, the modified sequence being a sequence in which 1 to 5 selected from C3, C2, I3, C1, and R4 of the DnaA boxes have been deleted, and a gyrase binding sequence.
(c) In one aspect, the circular DNA produced by the method of the present embodiment is the circular DNA of (a) or (b), in which the replication initiation sequence capable of binding to an enzyme that has a DnaA activity also has one or more of the following features (F1) to (F5); (F1) mutant DUE with a higher AT content than the wild type is present, (F2) the DUE has repetitive AT sequences in the M element, (F3) the DUE has four or more consecutive KAK motif sequences in the R element, (F4) the R5M sequence in the DOR region has a sequence identical to the consensus sequence of DnaA (TGTGNAWAA), and (F5) compared to the wild type, the DUE region has an increased number of TT[A/G]T(T) sequence motifs (for example, TTATT).
(d) In one aspect, the circular DNA produced by the method of the present embodiment includes a replication initiation sequence capable of binding to an enzyme having a DnaA activity, the replication initiation sequence having one or more of the following features (F1) to (F5); (F1) mutant DUE with a higher AT content than the wild type is present, (F2) the DUE has repetitive AT sequences in the M element, (F3) the DUE has four or more consecutive KAK motif sequences in the R element, (F4) the R5M sequence in the DOR region has a sequence identical to the consensus sequence of DnaA (TGTGNAWAA), and (F5) compared to the wild type, the DUE region has an increased number of TT[A/G]T(T) sequence motifs (for example, TTATT).
(e) In one aspect, the circular DNA produced by the method of the present embodiment is the circular DNA of any of (a) to (d), further including one or a pair of ter sequences inserted outward with respect to the replication initiation sequence.
(f) In one aspect, the circular DNA produced by the method of the present embodiment is the circular DNA of (d), in which the ter sequence is not adjacent to the replication initiation sequence.
(g) In one aspect, the circular DNA produced by the method of the present embodiment is the circular DNA of (a) or (b), in which the replication initiation sequence and the gyrase binding sequence are adjacent to each other, and the circular DNA further includes one or a pair of ter sequences inserted outward with respect to these sequences.
(h) In one aspect, the circular DNA produced by the method of the present embodiment is the circular DNA of (a) or (b), further including one or a pair of ter sequences inserted outward with respect to the replication initiation sequence, in which the distance between the gyrase binding sequence and the replication initiation sequence is greater than the distance between the ter sequence and the replication initiation sequence.
(i) In one aspect, the circular DNA produced by the method of the present embodiment is circular DNA of any of (e) to (h), in which the ter sequence is a mutant ter sequence.
(j) In one aspect, the circular DNA produced by the method of the present embodiment is circular DNA of any of (e) to (h), in which the ter sequence includes the sequence of SEQ ID NO: 5, 43, or 44.
(k) In one aspect, the circular DNA produced by the method of the present embodiment is the circular DNAs of any of (a) to (j) which does not include a DnaA box cluster at a different position from the replication initiation sequence.
(l) In one aspect, the circular DNA produced by the method of the present embodiment is the circular DNA of any of (a) to (j) which does not include a DnaA box cluster.

Does not include a DnaA box cluster.
(m) In one aspect, the circular DNA produced by the method of the present embodiment is the circular DNA of any of (a) to (j) which does not have, at a different position from the replication initiation sequence, a region within 60 bases (for example, within 50 bases) that includes the DnaAB consensus sequence 1 or an analogous sequence thereof, the DnaAB consensus sequence 2 or an analogous sequence thereof, and the DnaAB consensus sequence 2 or an analogous sequence thereof in this order.
(n) In one aspect, the circular DNA produced by the method of the present embodiment is the circular DNA of any of (a) to (j) which does not have, at a different position from the replication initiation sequence, a region within 40 bases (for example, within 30 bases) that includes the DnaAB consensus sequence 1 or an analogous sequence thereof and the DnaAB consensus sequence 2 or an analogous sequence thereof in this order.
(o) In one aspect, the circular DNA produced by the method of the present embodiment is the circular DNA of any of (a) to (j) which does not have a region within 60 bases (for example, within 50 bases) that includes the DnaAB consensus sequence 1 or an analogous sequence thereof, the DnaAB consensus sequence 2 or an analogous sequence thereof, and the DnaAB consensus sequence 2 or an analogous sequence thereof in this order.
(p) In one aspect, the circular DNA produced by the method of the present embodiment is the circular DNA of any of (a) to (j) which does not have a region within 40 bases (for example, within 30 bases) that includes the DnaAB consensus sequence 1 or an analogous sequence thereof and the DnaAB consensus sequence 2 or an analogous sequence thereof in this order.

The circular DNA prepared as described above is amplified by the RCR method. More specifically, the circular DNA is amplified by forming a reaction mixture of the circular DNA and a reaction liquid including
a first enzyme group that catalyzes replication of the circular DNA,
a second enzyme group that synthesizes two sister circular DNAs forming a catenane, and
a third enzyme group that catalyzes a separation reaction of the two sister circular DNAs, and
incubating the reaction mixture at a temperature in the range of 80°C or lower.

As the first enzyme group that catalyzes replication of the circular DNA, for example, the enzyme group described in Kaguni JM & Komberg A. Cell. 1984, 38:183-90 can be used. Specifically, examples of the first enzyme group include the following: one or more enzymes or enzyme groups selected from the group consisting of an enzyme having a DnaA activity, one or more nucleoid proteins, an enzyme or enzyme group having a DNA gyrase activity, a single-strand binding protein (SSB), an enzyme having a DnaB-type helicase activity, an enzyme having a DNA helicase loader activity, an enzyme having a DNA primase activity, an enzyme having a DNA clamp activity, and an enzyme or enzyme group having a DNA polymerase III* activity, or all combinations of the enzymes or enzyme groups. In one aspect, the first enzyme group includes an enzyme having a DnaA activity, a single-strand binding protein (SSB), an enzyme having a DnaB-type helicase activity, an enzyme having a DNA helicase loader activity, an enzyme having a DNA primase activity, an enzyme having a DNA clamp activity, and an enzyme or enzyme group having a DNA polymerase III* activity.

The biological origin of the enzyme having a DnaA activity is not particularly limited as long as the enzyme has an initiator activity similar to that of DnaA, which is an initiator protein in Escherichia coli. For example, DnaA derived from Escherichia coli can be suitably used. The DnaA derived from Escherichia coli may be contained in a reaction liquid as a monomer in the range of 1.0 nM to 10 µM, preferably in the range of 1.0 nM to 5.0 µM, 1.0 nM to 3 µM, 1 nM to 1.5 µM, 1.0 nM to 1.0 µM, 1.0 nM to 500 nM, 50 nM to 200 nM, or 50 nM to 150 nM, but the concentration is not limited thereto.

A nucleoid protein refers to a protein contained in a nucleoid. The biological origin of the one or more nucleoid proteins used in the present invention is not particularly limited as long as the proteins are enzymes having an activity similar to that of a nucleoid protein in Escherichia coli. For example, IHF derived from Escherichia coli, that is, a complex of IhfA and/or IhfB (heterodimer or homodimer), or HU derived from Escherichia coli, that is, a complex of hupA and hupB can be suitably used. The IHF derived from Escherichia coli may be contained in the reaction liquid as a heterodimer/homodimer in the range of 5 nM to 400 nM, preferably in the range of 5 nM to 200 nM, 5 nM to 100 nM, 5 nM to 50 nM, 10 nM to 50 nM, 10 nM to 40 nM, or 10 nM to 30 nM, but the concentration is not limited thereto. The HU derived from Escherichia coli may be contained in the reaction liquid in the range of 1 nM to 50 nM, preferably in the range of 5 nM to 50 nM or 5 nM to 25 nM, but the concentration is not limited thereto.

The biological origin of the enzyme or enzyme group having a DNA gyrase activity is not particularly limited as long as the enzyme has an activity similar to that of the DNA gyrase of Escherichia coli. For example, a complex of GyrA and GyrB derived from Escherichia coli can be suitably used. The complex of GyrA and GyrB derived from Escherichia coli may be contained in the reaction liquid as a heterotetramer in the range of 20 nM to 500 nM, preferably in the range of 20 nM to 400 nM, 20 nM to 300 nM, 20 nM to 200 nM, 50 nM to 200 nM, or 100 nM to 200 nM, but the concentration is not limited thereto.

The biological origin of the single-strand binding protein (SSB) is not particularly limited as long as the enzyme has an activity similar to that of the single-strand binding protein in Escherichia coli. For example, SSB derived from Escherichia coli can be suitably used. The SSB derived from Escherichia coli may be contained in the reaction liquid as a homotetramer in the range of 20 nM to 1000 nM, preferably in the range of 20 nM to 500 nM, 20 nM to 300 nM, 20 nM to 200 nM, 50 nM to 500 nM, 50 nM to 400 nM, 50 nM to 300 nM, 50 nM to 200 nM, 50 nM to 150 nM, 100 nM to 500 nM, or 100 nM to 400 nM, but the concentration is not limited thereto.

The biological origin of the enzyme having a DnaB-type helicase activity is not particularly limited as long as the enzyme has an activity similar to that of DnaB of Escherichia coli. For example, DnaB derived from Escherichia coli can be suitably used. The DnaB derived from Escherichia coli may be contained in the reaction liquid as a homohexamer in the range of 5 nM to 200 nM, preferably in the range of 5 nM to 100 nM, 5 nM to 50 nM, or 5 nM to 30 nM, but the concentration is not limited thereto.

The biological origin of the enzyme having a DNA helicase loader activity is not particularly limited as long as the enzyme has an activity similar to that of DnaC of Escherichia coli. For example, DnaC derived from Escherichia coli can be suitably used. The DnaC derived from Escherichia coli may be contained in the reaction liquid as a homohexamer in the range of 5 nM to 200 nM, preferably in the range of 5 nM to 100 nM, 5 nM to 50 nM, or 5 nM to 30 nM, but the concentration is not limited thereto.

The biological origin of the enzyme having a DNA primase activity is not particularly limited as long as the enzyme has an activity similar to that of DnaG of Escherichia coli. For example, DnaG derived from Escherichia coli can be suitably used. The DnaG derived from Escherichia coli may be contained in the reaction liquid as a monomer in the range of 20 nM to 1000 nM, preferably in the range of 20 nM to 800 nM, 50 nM to 800 nM, 100 nM to 800 nM, 200 nM to 800 nM, 250 nM to 800 nM, 250 nM to 500 nM, or 300 nM to 500 nM, but the concentration is not limited thereto.

The biological origin of the enzyme having a DNA clamp activity is not particularly limited as long as the enzyme has an activity similar to that of DnaN of Escherichia coli. For example, DnaN derived from Escherichia coli can be suitably used. The DnaN derived from Escherichia coli may be contained in the reaction liquid as a homodimer in the range of 10 nM to 1000 nM, preferably in the range of 10 nM to 800 nM, 10 nM to 500 nM, 20 nM to 500 nM, 20 nM to 200 nM, 30 nM to 200 nM, or 30 nM to 100 nM, but the concentration is not limited thereto.

The biological origin of the enzyme or enzyme group having a DNA polymerase III* activity is not particularly limited as long as the enzyme or enzyme group has an activity similar to that of a DNA polymerase III* complex of Escherichia coli. For example, an enzyme group including any of DnaX, HolA, HolB, HolC, HolD, DnaE, DnaQ, and HolE derived from Escherichia coli, preferably an enzyme group including a complex of DnaX, HolA, HolE, and DnaE derived from Escherichia coli, and more preferably an enzyme group including a complex of DnaX, HolA, HolB, HolC, HolD, DnaE, DnaQ, and HolE derived from Escherichia coli can be suitably used. The DNA polymerase III* complex derived from Escherichia coli may be contained in the reaction liquid as a heteromultimer in the range of 2 nM to 50 nM, preferably in the range of 2 nM to 40 nM, 2 nM to 30 nM, 2 nM to 20 nM, 5 nM to 40 nM, 5 nM to 30 nM, or 5 nM to 20 nM, but the concentration is not limited thereto.

In the present invention, the two sister circular DNAs forming a catenane refers to those in which two circular DNAs synthesized by a DNA replication reaction are linked to each other.

Examples of the second enzyme group that catalyzes an Okazaki fragment ligation reaction to synthesize two sister circular DNAs forming a catenane include one or more enzymes selected from the group consisting of an enzyme having a DNA polymerase I activity, an enzyme having a DNA ligase activity, and an enzyme having an RNase H activity, or a combination of the enzymes. In one aspect, the second enzyme group preferably includes an enzyme having a DNA polymerase I activity and an enzyme having a DNA ligase activity.

The biological origin of the enzyme having a DNA polymerase I activity is not particularly limited as long as the enzyme has an activity similar to that of DNA polymerase I of Escherichia coli. For example, DNA polymerase I derived from Escherichia coli can be suitably used. The DNA polymerase I derived from Escherichia coli may be contained in the reaction liquid as a monomer in the range of 10 nM to 200 nM, preferably in the range of 20 nM to 200 nM, 20 nM to 150 nM, 20 nM to 100 nM, 40 nM to 150 nM, 40 nM to 100 nM, or 40 nM to 80 nM, but the concentration is not limited thereto.

The biological origin of the enzyme having a DNA ligase activity is not particularly limited as long as the enzyme has an activity similar to that of DNA ligase of Escherichia coli. For example, DNA ligase derived from Escherichia coli or DNA ligase of T4 phage can be suitably used. The DNA ligase derived from Escherichia coli may be contained in the reaction liquid as a monomer in the range of 10 nM to 200 nM, preferably in the range of 15 nM to 200 nM, 20 nM to 200 nM, 20 nM to 150 nM, 20 nM to 100 nM, or 20 nM to 80 nM, but the concentration is not limited thereto.

The biological origin of the enzyme having an RNase H activity is not particularly limited as long as the enzyme has an activity of degrading the RNA strand of an RNA:DNA hybrid. For example, RNase H derived from Escherichia coli can be suitably used. The RNase H derived from Escherichia coli may be contained in the reaction liquid as a monomer in the range of 0.2 nM to 200 nM, preferably in the range of 0.2 nM to 200 nM, 0.2 nM to 100 nM, 0.2 nM to 50 nM, 1 nM to 200 nM, 1 nM to 100 nM, 1 nM to 50 nM, or 10 nM to 50 nM, but the concentration is not limited thereto.

As the third enzyme group that catalyzes a separation reaction of the two sister circular DNAs, the enzyme group described in, for example, Peng H & Marians KJ. PNAS. 1993, 90: 8571-8575 can be used. Specifically, examples of the third enzyme group include one or more enzymes selected from the group consisting of an enzyme having a topoisomerase IV activity, an enzyme having topoisomerase III activity, and an enzyme having a RecQ-type helicase activity, or a combination of the enzymes. In one aspect, the third enzyme group preferably includes an enzyme having a topoisomerase IV activity and/or an enzyme having a topoisomerase III activity.

The biological origin of the enzyme having a topoisomerase III activity is not particularly limited as long as the enzyme has an activity similar to that of topoisomerase III of Escherichia coli. For example, topoisomerase III derived from Escherichia coli can be suitably used. The topoisomerase III derived from Escherichia coli may be contained in the reaction liquid as a monomer in the range of 20 nM to 500 nM, preferably in the range of 20 nM to 400 nM, 20 nM to 300 nM, 20 nM to 200 nM, 20 nM to 100 nM, or 30 to 80 nM, but the concentration is not limited thereto.

The biological origin of the enzyme having a RecQ-type helicase activity is not particularly limited as long as the enzyme has an activity similar to that of RecQ of Escherichia coli. For example, RecQ derived from Escherichia coli can be suitably used. The RecQ derived from Escherichia coli may be contained in the reaction liquid as a monomer in the range of 20 nM to 500 nM, preferably in the range of 20 nM to 400 nM, 20 nM to 300 nM, 20 nM to 200 nM, 20 nM to 100 nM, or 30 to 80 nM, but the concentration is not limited thereto.

The biological origin of the enzyme having a topoisomerase IV activity is not particularly limited as long as the enzyme has an activity similar to that of topoisomerase IV of Escherichia coli. For example, topoisomerase IV derived from Escherichia coli, which is a complex of ParC and ParE, can be suitably used. The topoisomerase IV derived from Escherichia coli may be contained in the reaction liquid as a heterotetramer in the range of 0.1 nM to 50 nM, preferably in the range of 0.1 nM to 40 nM, 0.1 nM to 30 nM, 0.1 nM to 20 nM, 1 nM to 40 nM, 1 nM to 30 nM, 1 nM to 20 nM, 1 nM to 10 nM, or 1 nM to 5 nM, but the concentration is not limited thereto.

The reaction liquid may contain an additional enzyme. For example, when the circular DNA to be amplified by the RCR method has the ter sequence described above, the reaction liquid may further contain a protein having an activity of inhibiting replication by binding to the ter sequence (for example, a Tus protein derived from Escherichia coli).

The reaction liquid may further contain a linear DNA-specific exonuclease. The presence of the linear DNA-specific exonuclease in the reaction liquid has an effect of reducing the amount of linear DNA generated by a double-strand break or the like during the amplification reaction. thereby improving the yield of the target circular DNA.

The linear DNA-specific exonuclease is an enzyme that hydrolyzes sequentially from the 5' end or the 3' end of linear DNA. The type and biological origin of the linear DNA-specific exonuclease is not particularly limited as long as the linear DNA-specific exonuclease has an activity of hydrolyzing sequentially from the 5' end or the 3' end of linear DNA. For example, RecBCD (exonuclease V), λ exonuclease, exonuclease III, exonuclease VIII, T5 exonuclease, T7 exonuclease, or Plasmid-Safe (registered trademark) ATP-Dependent DNase (epicentre) can be used. The linear DNA exonuclease may be contained in the reaction liquid in the range of 0.001 to 1.0 U/µL, preferably in the range of 0.005 U to 1.0 U/µL, 0.01 to 1.0 U/µL, 0.05 to 1.0 U/µL, or 0.1 to 1.0 U/µL, but the concentration is not limited thereto. As for the enzyme activity unit (U) for the linear DNA exonuclease, 1 U is defined as the amount of enzyme required to render 1 nmol of deoxyribonucleotides in linear DNA acid-soluble in a reaction performed at 37°C for 30 minutes.

The reaction liquid may further contain a single-stranded DNA-specific exonuclease. The presence of the single-stranded DNA-specific exonuclease in the reaction liquid has an effect of reducing the amount of a low-molecular-weight secondary amplification product generated during the amplification reaction, thereby improving the yield of the target circular DNA.

The single-stranded DNA-specific exonuclease is an enzyme that sequentially hydrolyzes the nucleotides at the 5' end or the 3' end of single-stranded DNA. The type and biological origin of the single-stranded DNA-specific exonuclease is not particularly limited as long as the single-stranded DNA-specific exonuclease has an activity of sequentially hydrolyzing the nucleotides at the 5' end or the 3' end of single-stranded DNA. For example, exonuclease I (exo I), RecJ, exonuclease T, or the like can be used. A preferred single-stranded DNA-specific exonuclease is exo I. The single-stranded DNA-specific exonuclease may be contained in the reaction liquid in the range of 0.1 to 1.0 U/µL, preferably in the range of 0.15 to 1.0 U/µL, 0.2 to 1.0 U/µL, or 0.2 to 0.5 U/µL, but the concentration is not limited thereto. As for the enzyme activity unit (U) for exo I, 1 U is defined as the amount of enzyme required to render 10 nmol of deoxyribonucleotides in single-stranded DNA acid-soluble in a reaction performed at 37°C for 30 minutes. As for the enzyme activity unit (U) for RecJ, 1 U is defined as the amount of enzyme required to render 0.05 nmol of deoxyribonucleotides in single-stranded DNA acid-soluble in a reaction performed at 37°C for 30 minutes.

As enzymes contained in the reaction liquid containing the first, second, and third enzyme groups, commercially available enzymes may be used, or enzymes extracted from microorganisms or the like and purified as necessary may be used. Extraction and purification of enzymes from microorganisms can be performed as appropriate using techniques available to those skilled in the art.

When enzymes other than the enzymes derived from Escherichia coli described above are used as the first, second, and third enzyme groups, the enzymes can be used within concentration ranges corresponding in terms of enzyme activity units to the concentration ranges specified for the enzymes derived from Escherichia coli described above.

The reaction liquid may contain a buffer, ATP, GTP, CTP, UTP, dNTP, a magnesium ion source, and an alkali metal ion source.

The buffer contained in the reaction liquid is not particularly limited as long as the buffer is suitable for use at pH 7 to 9, preferably at pH 8. Examples of such a buffer include Tris-HCl, Tris-OAc, Hepes-KOH, a phosphate buffer, MOPS-NaOH, and Tricine-HCl. A preferred buffer is Tris-HCl or Tris-OAc. The concentration of the buffer can be selected as appropriate by those skilled in the art and is not particularly limited. In the case of Tris-HCl or Tris-OAc, the concentration of, for example, 10 mM to 100 mM, 10 mM to 50 mM, or 20 mM can be selected.

ATP refers to adenosine triphosphate. The concentration of ATP contained in the reaction liquid at the start of the reaction may be, for example, in the range of 0.1 mM to 3 mM, preferably in the range of 0.1 mM to 2 mM, 0.1 mM to 1.5 mM, or 0.5 mM to 1.5 mM.

GTP, CTP, and UTP refer to guanosine triphosphate, cytidine triphosphate, and uridine triphosphate, respectively. The concentrations of GTP, CTP, and UTP contained in the reaction liquid at the start of the reaction may each independently be, for example, in the range of 0.1 mM to 3.0 mM, preferably in the range of 0.5 mM to 3.0 mM or 0.5 mM to 2.0 mM.
dNTP is the collective term for deoxyadenosine triphosphate (dATP), deoxyguanosine triphosphate (dGTP), deoxycytidine triphosphate (dCTP), and deoxythymidine triphosphate
(dTTP). The concentration of dNTP contained in the reaction liquid at the start of the reaction may be, for example, in the range of 0.01 to 1 mM, preferably in the range of 0.05 mM to 1 mM or 0.1 mM to 1 mM.

The magnesium ion source is a substance that provides magnesium ions (Mg2+) to the reaction liquid. Examples of the magnesium ion source include Mg(OAc)2, MgCl2, and MgSO4. A preferred magnesium ion source is Mg(OAc)2. The concentration of the magnesium ion source contained in the reaction liquid at the start of the reaction may be, for example, a concentration that provides magnesium ions to the reaction liquid in the range of 5 to 50 mM.

The alkali metal ion source is a substance that provides alkali metal ions to the reaction liquid. Examples of the alkali metal ions include sodium ions (Na+) and potassium ions (K+). Examples of the alkali metal ion source include potassium glutamate, potassium aspartate, potassium chloride, potassium acetate, sodium glutamate, sodium aspartate, sodium chloride, and sodium acetate. A preferred alkali metal ion source is potassium glutamate or potassium acetate. The concentration of the alkali metal ion source contained in the reaction liquid at the start of the reaction may be a concentration that provides alkali metal ions to the reaction liquid at 100 mM or more, preferably in the range of 100 mM to 300 mM, but the concentration is not limited thereto.

The reaction liquid can further contain a non-specific protein adsorption inhibitor (bovine serum albumin, lysozyme, gelatin, heparin, casein, or the like), a non-specific nucleic acid adsorption inhibitor (transfer RNA (tRNA), ribosomal RNA (rRNA), messenger RNA (mRNA), glycogen, heparin, oligo DNA, polyinosine-polycytidine (poly(I-C)), polydeoxyinosine-polydeoxycytidine (poly(dI-dC)), polyadenine (poly(A)), polydeoxyadenine (poly(dA)), or the like), a linear DNA-specific exonuclease (RecBCD, λ exonuclease, exonuclease III, exonuclease VIII, T5 exonuclease, T7 exonuclease, Plasmid-Safe (registered trademark) ATP-Dependent DNase (epicentre), or the like), a RecG-type helicase (RecG derived from Escherichia coli or the like), an ammonium salt (ammonium sulfate, ammonium chloride, ammonium acetate, or the like), and a reducing agent (DTT, β-mercaptoethanol, glutathione, or the like), and when the reaction liquid contains DNA ligase derived from Escherichia coli, the reaction liquid may contain nicotinamide adenine dinucleotide (NAD), which is a cofactor of the DNA ligase.

A reaction mixture for amplifying circular DNA may be formed by mixing a reaction liquid containing the enzyme and a cell-free protein expression system directly with the circular DNA serving as a template. The cell-free protein expression system may be a cell-free translation system in which total RNA containing RNA consisting of a sequence complementary to the base sequence of the gene encoding the enzyme, mRNA, an in vitro transcription product, or the like is used as template RNA, or a cell-free transcription and translation system in which the gene encoding each enzyme, an expression vector containing the gene encoding each enzyme, or the like is used as template DNA.

The reaction liquid is mixed with circular DNA to form a reaction mixture, and the reaction mixture is incubated at a temperature in the range of 80°C or lower, thereby obtaining a reaction product that includes the circular DNA, which has been amplified. The amplification may be isothermal or non-isothermal amplification.

In the case of isothermal amplification, the isothermal condition is not particularly limited, as long as the DNA amplification reaction or the DNA replication reaction can proceed. For example, the isothermal condition can be a constant temperature within the optimum temperature range for the DNA polymerase. Examples of the isothermal condition include a constant temperature of 15°C or higher, 16°C or higher, 18°C or higher, 20°C or higher, 23°C or higher, 24°C or higher, 25°C or higher, 26°C or higher, 27°C or higher, or 30°C or higher, and a constant temperature of 80°C or lower, 75°C or lower, 70°C or lower, 65°C or lower, 60°C or lower, 50°C or lower, 45°C or lower, 40°C or lower, 35°C or lower, or 33°C or lower. The isothermal condition can also be, for example, a constant temperature within the range of 15°C to 80°C, 16°C to 80°C, 18°C to 80°C, 20°C to 80°C, or 25°C to 80°C, a constant temperature within the range of 15°C to 75°C, 16°C to 75°C, 18°C to 75°C, 20°C to 75°C, or 25°C to 75°C, a constant temperature within the range of 15°C to 70°C, 16°C to 70°C, 18°C to 70°C, 20°C to 70°C, or 25°C to 70°C, a constant temperature within the range of 15°C to 65°C, 16°C to 65°C, 18°C to 65°C, 20°C to 65°C, or 25°C to 65°C, a constant temperature within the range of 25°C to 50°C, a constant temperature within the range of 25°C to 40°C, a constant temperature within the range of 30°C to 33°C, or about 30°C. As used herein, the term "isothermal" means maintaining the temperature within the range of ±7°C, ±5°C, ±3°C, or ±1°C relative to the temperature set during the reaction. The reaction time for the isothermal amplification can be set as appropriate in accordance with the amount of the amplification product of the target circular DNA, for example, 1 hour to 30 hours, preferably 2 hours to 25 hours, more preferably 3 hours to 20 hours, even more preferably 5 hours to 15 hours. In one aspect, the circular DNA of the present embodiment allows a high amplification concentration to be obtained even in a short reaction time.

When the circular DNA is amplified non-isothermally, the circular DNA can be amplified under a temperature condition in which the incubation is performed under temperature cycles repeating incubation at two temperatures of 80°C or lower, preferably 65°C or lower. A first temperature of the temperature cycle is the temperature at which the replication of double-stranded DNA can be initiated, and a second temperature is the temperature at which the initiation of the replication is suppressed, and the DNA elongation reaction proceeds. The first temperature can be 30°C or higher, for example, 30°C to 80°C, 30°C to 50°C, 30°C to 40°C, or 37°C. The incubation time at the first temperature is not particularly limited, and it may be 10 seconds to 10 minutes per cycle, with 1 minute being preferred. The second temperature can be 27°C or lower, for example, 10°C to 27°C, 16°C to 25°C, or 24°C. The incubation time at the second temperature is not particularly limited, but is preferably set according to the length of the circular DNA to be amplified. For example, the incubation may be performed for 1 to 10 seconds per 1000 bases per cycle. The number of the temperature cycles is not particularly limited, and it may be 10 to 50 cycles, 20 to 45 cycles, 25 to 45 cycles, or 40 cycles.

The method of the present embodiment may further include, after incubating the reaction mixture at a temperature in the range of 80°C or lower to obtain the reaction product that includes the circular DNA which has been amplified, diluting the reaction product two-fold or more, preferably 3-fold, 4-fold, or 5-fold or more, with a solution that does not contain the first to third enzyme groups, and then maintaining (re-incubating) the diluted product. Without being bound by theory, while new replication initiation is suppressed by the dilution of the enzyme groups, the ongoing replication elongation, catenane formation, and separation reaction continue to proceed due to the effect of the residual enzymes. In addition, by-products generated during the reaction by a nick or the like can be repaired by the effect of residual ligase and the like in this process. Therefore, the transition from the amplification intermediate or the byproduct to the final product is specifically induced, and improvement in the yield of the target circular DNA can be expected.

The maintenance of the diluted product after the dilution can be performed under conditions in which the second enzyme group and the third enzyme group act, for example, at 15 to 50°C, preferably at 20°C to 40°C, more preferably at 25°C to 35°C, for 10 minutes to 3 hours, preferably for 15 minutes to 2 hours, more preferably for 20 minutes to 1 hour.

The composition of the solution that does not contain the first third enzyme groups is not particularly limited as long as it is a solution in which the second enzyme group and the third enzyme group can act. Conveniently, a solution having the same composition as the reaction liquid described above, except for being free of the first, second, and third enzyme groups, can be used. Such a reaction liquid may contain a buffer, ATP, GTP, CTP, UTP, dNTP, a magnesium ion source, and an alkali metal ion source. The solution may further contain one or more selected from the non-specific protein adsorption inhibitor, the non-specific nucleic acid adsorption inhibitor, the ammonium salt, the reducing agent, and NAD described above.

In one aspect, when the replication initiation sequence, which is capable of binding to an enzyme that has a DnaA activity, in the circular DNA has mutant DUE with a higher AT content than the wild type, it is preferable that the method of the present embodiment further includes diluting the reaction product two-fold or more with a solution not containing the first to third enzyme groups, and then maintaining (re-incubating) the diluted product.

The present embodiment also relates to circular DNA including a replication initiation sequence capable of binding to an enzyme that has a DnaA activity and a gyrase binding sequence, the circular DNA not including a DnaA box cluster at a different position from the replication initiation sequence. The present embodiment also relates to circular DNA including a replication initiation sequence capable of binding to an enzyme that has a DnaA activity, a gyrase binding sequence, and a plasmid replication origin, in which the plasmid replication origin does not include a DnaA box cluster. The circular DNA may have one or a pair of ter sequences. The replication initiation sequence of the circular DNA, the replication initiation sequence being capable of binding to the enzyme that has the DnaA activity, may be mutant DUE. Regarding each sequence, the description in the method for producing the circular DNA above can be referred to. Such circular DNA can be efficiently amplified by the RCR method.

In one aspect of the present embodiment, the circular DNA serving as a template for the method of the present embodiment is conveniently created by preparing a DNA cassette including (i) a replication initiation sequence capable of binding to an enzyme that has a DnaA activity and (ii) a gyrase binding sequence, a DNA cassette including (i) a replication initiation sequence capable of binding to an enzyme that has a DnaA activity and having mutant DUE with a higher AT content than the wild type, a DNA cassette including (i) a replication initiation sequence capable of binding to an enzyme that has a DnaA activity and (ii) a gyrase binding sequence and having mutant DUE with a higher AT content than the wild type, or any of these DNA cassettes having one or a pair of ter sequences, and ligating a DNA fragment or circular DNA not including a DnaA box cluster and the DNA cassette.

The DNA fragment or circular DNA not including a DnaA box cluster may be derived from a commercially available plasmid, for example, a plasmid having p15A or fori as a plasmid replication origin or linear DNA obtained by enzymatically cleaving the plasmid.

The method for ligating the DNA fragment or circular DNA not including a DnaA box cluster and the DNA cassette is not particularly limited. As the ligation reaction between linear DNA fragments, for example, methods using a commercially available kit, such as the In-Fusion method, the Gibson Assembly method, the Golden Gate Assembly method, and USER (registered trademark) Cloning (manufactured by NEB), the Recombination Assembly method (WO 2019/009361 A), and the like are known, and such known techniques can be used.

As the ligation reaction between linear DNA and circular DNA, for example, Gateway cloning using a site-specific recombination mechanism is known, and a reagent kit is commercially available (manufactured by Thermo Fisher Scientific Inc.). Alternatively, a method using a homologous recombination reaction carried out using a RecA family recombinase and an exonuclease, if necessary (WO 2023/028145 A), may be used.

### Examples

Next, the present invention will be described in more detail with reference to Examples and the like, but the present invention is not limited to these examples.

### Preparation Example 1

The following sequence having ter sequences adjacent to both ends of a portion (264 bases) of an oriC sequence derived from an Escherichia coli chromosome was prepared as the OriC1.0 cassette (SEQ ID NO: 3). The underlined portions are the ter sequences (SEQ ID NO: 1), and the portion sandwiched between the ter sequences is the oriC sequence (SEQ ID NO: 2).

**[Table 3]**

| OriC1.0 cassette | SEQ ID NO |
|---|---|
| AGTATGTTGTAACTAAAGTATTAAAAAGAAGATCTATTTATTTAGAGATCTGTTCTATTGTGATCTCTTATTAGG ATCGCACTGCCCTGTGGATAACAAGGATCCGGCTTTTAAGATCAACAACCTGGAAAGGATCATTAACTGTGAATG ATCGGTGATCCTGGACCGTATAAGCTGGGATCAGAATGAGGGGTTATACACAACTCAAAAACTGAACAACAGTTG TTCTTTGGATAACTACCGGTTGATCCAAGCTTCCTGACAGAGTTATCCACAGTAGATCTTTAGTTACAACATACT | 3 |

### [Preparation Example 2]

A DNA fragment in which a mutant ter sequence (terG16 sequence, SEQ ID NO: 5) was ligated adjacent to the 5' side of an oriC sequence derived from an Escherichia coli chromosome (SEQ ID NO: 4), a sequence complementary to the terG16 sequence was ligated near the 3' side of the oriC, a Tac promoter sequence (SEQ ID NO: 6) was located nearer to the 5' side of the terG16 sequence, and the terminator sequence (SEQ ID NO: 7) of the Escherichia coli fdhF gene was located nearer to the 3' side of the sequence complementary to the terG16 sequence was prepared as the OriC2.0 cassette (426 bp, SEQ ID NO: 8). The terG16 sequence is the base sequence of SEQ ID NO: 37 with a single base mutation. The underlined portions are the terG16 sequences, and the portion indicated by uppercase letters in the region sandwiched between the underlined portions is the oriC sequence (286 bp).

**[Table 4]**

| OriC2.0 cassette | SEQ ID NO |
|---|---|
| GAGCTGTTGACAGAGGGTCATTTTCACACTATAATGCAGTGAATCCCAAACAGTATGTTGTACCTAAAGGGATCC TGGGTATTAAAAAGAAGATCTATTTATTTAGAGATCTGTTCTATTGTGATCTCTTATTAGGATCGCACTGCCCTG TGGATAACAAGGATCCGGCTTTTAAGATCAACAACCTGGAAAGGATCATTAACTGTGAATGATCGGTGATCCTGG ACCGTATAAGCTGGGATCAGAATGAGGGGTTATACACAACTCAAAAACTGAACAACAGTTGTTCTTTGGATAACT ACCGGTTGATCCAAGCTTCCTGACAGAGTTATCCACAGTAGATCGCACGATCTGTcagctcatttcCTTTAGGTA CAACATACTAGAATATTTGCCTACAGCCTCCTTTCGGAGGCTGTTTTTTA | 8 |

### [Preparation Example 3]

A DNA fragment in the OriC2.0 cassette sequence having SGS (SEQ ID NO: 9) ligated to the 5' side of the oriC was prepared as the OriCsg cassette (582 bp, SEQ ID NO: 10). The base sequence is shown in Table 5. In the table, the region indicated by underlined uppercase letters represents the SGS (the bold letters being the consensus sequence of SGS), and the region near the center indicated by uppercase letters (without underline) represents the oriC sequence. The regions enclosed by boxes represent the terG16 sequence and the sequence complementary thereto.

**[Table 5]**

| OriCsg cassette | SEQ ID NO |
|---|---|
| GAGCTGTTGACAGAGGGTCATTTTCACACTATAATGCAGTGAATCCCAAACAGTATGTTGTACCTAAAGggacct gTTTTTGATGCCATCAGAAAACGCGTCAGCGCCGCTCTGAGGCAATAAACAGAATCAGGCATAAAATCAGCCGCA CAGATTTTTTAAAACGCGCCACGGGATTTTTAAACCGGTATTTAACGGTGTATGAATCCCGTTTTATCTTCCTTT GGATCCTGGGTATTAAAAAGAAGATCTATTTATTTAGAGATCTGTTCTATTGTGATCTCTTATTAGGATCGCACT GCCCTGTGGATAACAAGGATCCGGCTTTTAAGATCAACAACCTGGAAAGGATCATTAACTGTGAATGATCGGTGA TCCTGGACCGTATAAGCTGGGATCAGAATGAGGGGTTATACACAACTCAAAAACTGAACAACAGTTGTTCTTTGG ATAACTACCGGTTGATCCAAGCTTCCTGACAGAGTTATCCACAGTAGATCGCACGATCTGTcagctcatttcCTT TAGGTACAACATACTAGAATATTTGCCTACAGCCTCCTTTCGGAGGCTGTTTTTTA | 10 |

### [Preparation Example 4]

A DNA fragment in the OriC2.0 cassette sequence having a Stuffer sequence derived from Escherichia coli (a sequence having no function, which is located outside the oriC of the Escherichia coli genome) ligated to the 5' side of the oriC was prepared as an OriCsg_Stuffer cassette (582 bp, SEQ ID NO: 11). The base sequence is shown in Table 6. In the table, the region indicated by underlined uppercase letters represents the Stuffer sequence, and the region near the center indicated by uppercase letters (without underline) represents the oriC sequence. The regions enclosed by boxes represent the terG16 sequence and the sequence complementary thereto.

**[Table 6]**

| OriCsg_Stuffer cassette | SEQ ID NO |
|---|---|
| GAGCTGTTGACAGAGGGTCATTTTCACACTATAATGcagtgaatcccaaacAGTATGTTGTACCTAAAGggacct gCCTCGGTGCCTGCATGACCCCCGCCAATGATGATGACGTCAAAAGGATCCGGATAAAACATGGTGATTGCCTCG CATAACGCGGTATGAAAATGGATTGAAGCCCGGGCCGTGGATTCTACTCAACTTTGTCGGCTTGAGAAAGACCTG GGATCCTGGGTATTAAAAAGAAGATCTATTTATTTAGAGATCTGTTCTATTGTGATCTCTTATTAGGATCGCACT GCCCTGTGGATAACAAGGATCCGGCTTTTAAGATCAACAACCTGGAAAGGATCATTAACTGTGAATGATCGGTGA TCCTGGACCGTATAAGCTGGGATCAGAATGAGGGGTTATACACAACTCAAAAACTGAACAACAGTTGTTCTTTGG ATAACTACCGGTTGATCCAAGCTTCCTGACAGAGTTATCCACAGTAGATCGCACGATCTGTcagctcatttcCTT TAGGTACAACATACTagaatatttgccTACAGCCTCCTTTCGGAGGCTGTTTTTTTA | 11 |

### [Preparation Example 5]

A DNA fragment in the OriC2.0 cassette sequence having mutant DUE in which the M and R elements among the L, M, and R elements constituting the DUE of the oriC had higher AT contents than those in the wild type was prepared as an OriC5.6AT cassette (427 bp, SEQ ID NO: 12). The base sequence is shown in Table 7. In the table, the portion indicated by bold letters is the DUE portion, and the portions where the bold letters are enclosed by boxes are the mutated portions in the mutant DUE. The underlined portions are the M element (underlined portion on the 5' end side) and the R element (underlined portion on the 3' end side; has a single-base deletion mutation) of the DUE.

**[Table 7]**

| OriC5 6AT cassette | SEQ ID NO |
|---|---|
| GAGCTGTTGACAGAGGGTCATTTTCACACTATAATGccgctatagtgagtcgtAGTATGTTGTACCTAAAGGG ATCCTGGGTATTAAAAAGAAGATCTATTTATTTATATATATATTATATTATTTAT_TATTATTAGGATCGCAC TGCCCTGTGGATAACAAGGATCCGGCTTTTAAGATCAACAACCTGGAAAGGATCATTAACTGTGAATGATCGGT GATCCTGGACCGTATAAGCTGGGATCAGAATGAGGGGTTATACACAGCTGAGGAACTGAAGAGCAGTTGTTCTT TGGATAACTACCGGTTGATCCAAGCTTCCTGACAGAGTTATCCACAGTAGATCGCACGATCTGtcagctcattt cCTTTAGGTACAACATACtagaatatttgccTACAGCCTCCTTTCGGAGGCTGTTTTTTTA | 12 |

### [Preparation Example 6]

The following four types of plasmids were prepared.
·E1-p15A plasmid: E1 sequence (579 bp) including a marker gene was ligated to pACYC plasmid (Nippon Gene Co., Ltd.) by the USER method described later, and amplified by RCR amplification reaction to create a plasmid with a total length of 3.5 kbp.
·H2-p15A plasmid: H2 sequence (sequence encoding a part of a known virus, 3.8 kbp) was ligated to pACYC plasmid (Nippon Gene Co., Ltd.) 3.8kbp) by the USER method described later, and amplified by RCR amplification reaction to create a plasmid with a total length of 6.8 kbp.

·pUC plasmid: E1 sequence (579 bp) was ligated to plasmid pUC4K (GenBank accession number: X06404, total length: 3.9kbp) by the USER method described later, and amplified by RCR amplification reaction to create a plasmid with a total length of 3.5 kbp.
·pBeloBAC plasmid: Manufactured by NEB (GenBank accession number: U51113, total length: 7.5 kb)

### [Example 1] Ligation and amplification of p15A plasmid and OriC cassette

The fragments of each of the OriC cassettes from Preparation Examples 1 to 5 and some fragments of the two types of p15A plasmids from Preparation Example 6 were each prepared by PCR using the primers listed in Table 8 containing dUTP. In Table 8, u represents deoxyuridine (dU). Each of the DNA fragments prepared was mixed, added to 5 µL of a reaction liquid (1x rCutSmart buffer (manufactured by NEB), 7.5% by mass PEG8000, and 10 mU/µL Thermolabile USER II Enzyme (manufactured by NEB, an enzyme which contains uracil DNA glycosylase and endonuclease VIII and generates a single base gap at the dUTP moiety), and ligated by being treated at 37°C for 15 minutes and at 60°C for 5 minutes (USER method). Then, the target circular DNA was amplified by the RCR amplification reaction using the ligated DNA as the template.

**[Table 8]**

| Primer name | Sequence | SEQ ID NO |
|---|---|---|
| OriC1.0_FW | atgccgcatagtatgttgtaactaaagatctactgtg | 13 |
| OriC1.0_RV_dU | gtgtcggggcuggcttaacagtatgtugtaactaaagtatuaaaaa g | 14 |
| OriC_FW_1 (OriC2. 0_FW/Or iCsg_FW/Or iCsg Stuffer _FW/Or iC5. 6AT_FW) | atgccgcattaaaaaaacagcctccgaaaggag | 15 |
| OriC_RV_1 (OriC2.0_RV_dU/OriCsg_RV_dU/ OriCsg_Stuffer_RV_dU/OriC5. 6 AT_RV_dU) | aacgagctgtugacagagggucattttc | 16 |
| Kanamyc i n_FW_dU | atcccttcagagucccggguag | 17 |
| Kanamyc i n_RV | aggctgtttttttaatgcggcatcagagcag | 18 |
| E1-p15A_FW_dU | accctctgucaacagctcgtuaagccagccccgacac | 19 |
| H2-p15A_FW_dU | | |
| E1-p15A_RV_dU | actcagactttatucaaagaccac | 20 |
| H2-p15A_FW_dU | | |

The RCR amplification reaction was performed as follows. An RCR amplification reaction solution (5 µL) was prepared by mixing 0.5 µL of a solution containing the template with 4.5 µL of a reaction mixture having the composition shown in Table 9 (diluted as appropriate), and the RCR amplification reaction solution was incubated at 33°C for 10 hours to perform RCR amplification reaction, and then cooled to 4°C.

**[Table 9]**

| Reaction mixture | |
|---|---|
| Reaction buffer | |
| Tris-HC I (pH8. 0) | 20 mM |
| Dithiothreitol | 8 mM |
| Potassium glutamate | 150 mM |
| Mg(OAc)₂ | 10 mM |
| Creatine phosphate | 4 m M |
| ATP | 1 mM |
| GTP, CTP. UTP | 1 mM each |
| dNTPs | 0.1 mM each |
| tRNA | 50 ng/µL |
| NAD | 0.25 mM |
| Ammonium sulfate | 10 mM |
| Bovine serum albumin (BSA) | 0.5 m g / mL |
| Creatine kinase | 20 ng/µL |

| Enzyme group | |
|---|---|
| SSB | 400 nM |
| IHF | 20 nM |
| DnaG | 400n M |
| DnaN | 40 nM |
| PolIII* | 5 nM |
| DnaB, DnaC | 20 nM |
| DnaA | 100 nM |
| RNaseH | 10 nM |
| Ligase | 50 n M |
| PolI | 50 nM |
| GyrA, GyrB | 50 nM |
| Topo IV | 5 n M |
| Topo III | 50 nM |
| RecQ | 50 nM |
| Tus | 60 nM |

In Table 9, SSB represents SSB derived from Escherichia coli, IHF represents a complex of IhfA and IhfB derived from Escherichia coli, DnaG represents DnaG derived from Escherichia coli, DnaN represents DnaN derived from Escherichia coli, Pol III* represents a DNA polymerase III* complex, which is a complex of DnaX, HolA, HolB, HolC, HolD, DnaE, DnaQ, and HolE derived from Escherichia coli, DnaB represents DnaB derived from Escherichia coli, DnaC represents DnaC derived from Escherichia coli, DnaA represents DnaA derived from Escherichia coli, RNase H represents RNase H derived from Escherichia coli, Ligase represents DNA ligase derived from Escherichia coli, Pol I represents DNA polymerase I derived from Escherichia coli, GyrA represents GyrA derived from Escherichia coli, GyrB represents GyrB derived from Escherichia coli, Topo IV represents a complex of ParC and ParE derived from Escherichia coli, Topo III represents topoisomerase III derived from Escherichia coli, and RecQ represents RecQ derived from Escherichia coli.

The SSB was prepared by purifying SSB from an Escherichia coli strain expressing SSB through a process including ammonium sulfate precipitation and ion exchange column chromatography.

IHF was prepared by purifying IhfA and IhfB from an Escherichia coli strain coexpressing IhfA and IhfB through a process including ammonium sulfate precipitation and affinity column chromatography.

DnaG was prepared by purifying DnaG from an Escherichia coli strain expressing DnaG through a process including ammonium sulfate precipitation, anion exchange column chromatography, and gel filtration column chromatography.

DnaN was prepared by purifying DnaN from an Escherichia coli strain expressing DnaN through a process including ammonium sulfate precipitation and anion exchange column chromatography.

Pol III* was prepared by purifying DnaX, HolA, HolE, HolC, HolD, DnaE, DnaQ, and HolE from an Escherichia coli strain coexpressing DnaX, HolA, HolB, HolC, HolD, DnaE, DnaQ, and HolE through a process including ammonium sulfate precipitation, affinity column chromatography, and gel filtration column chromatography.

DnaB and DnaC were prepared by purifying DnaB and DnaC from an Escherichia coli strain coexpressing DnaB and DnaC through a process including ammonium sulfate precipitation, affinity column chromatography, and gel filtration column chromatography.

DnaA was prepared by purifying DnaA from an Escherichia coli strain expressing DnaA through a process including ammonium sulfate precipitation, dialysis precipitation, and gel filtration column chromatography.

GyrA and GyrB were prepared by purifying GyrA and GyrB from a mixture of an Escherichia coli strain expressing GyrA and an Escherichia coli strain expressing GyrB through a process including ammonium sulfate precipitation, affinity column chromatography, and gel filtration column chromatography.

Topo IV was prepared by purifying ParC and ParE from a mixture of an Escherichia coli strain expressing ParC and an Escherichia coli strain expressing ParE through a process including ammonium sulfate precipitation, affinity column chromatography, and gel filtration column chromatography.

Topo III was prepared by purifying Topo III from an Escherichia coli strain expressing Topo III through a process including ammonium sulfate precipitation and affinity column chromatography.

RecQ was prepared by purifying RecQ from an Escherichia coli strain expressing RecQ through a process including ammonium sulfate precipitation, affinity column chromatography, and gel filtration column chromatography.

As for RNase H, Ligase, and Pol I, commercially available enzymes derived from Escherichia coli (Takara Bio Inc.) were used.

Tus was prepared by purifying Tus from an Escherichia coli strain expressing Tus through a process including affinity column chromatography and gel filtration column chromatography.

### [Example 2] Ligation and amplification of pUC plasmid and OriC cassette

The fragments of each of the OriC cassettes from Preparation Examples 1 to 3 and some fragments of the pUC plasmid from Preparation Example 6 were each prepared by PCR using the primers containing dUTP. As the primers, those shown in Example 1 and those listed in Table 10 were used. Each of the DNA fragments prepared was mixed and ligated by the same method as in Example 1. Using the same method as in Example 1, the target circular DNA was amplified by the RCR amplification reaction using the ligated DNA as the template.

**[Table 10]**

| Primer name | Sequence | SEQ ID NO |
|---|---|---|
| pUCori_FW | gttaagccagccocgacacttgagatcctttttttctgcgcgtaatc | 21 |
| pUCori_RV | ctggcgtttttccataggctc | 22 |

### [Example 3] Ligation and amplification of pBeloBAC plasmid and OriC cassette

The fragments of each of the OriC cassettes from Preparation Examples 1 to 3 and 5 and some fragments of the pBeloBAC plasmid from Preparation Example 6 were each prepared by PCR using the primers listed in Table 11. For a homologous recombination reaction, 200 pM plasmid and 200 pM fragments of each OriC cassette to which an overlapping sequence was added were added to 5 µL of a reaction liquid (1 µM RecA, 80 mU/µL exonuclease III, 20 mM Tris-HCl (pH 8.0), 4 mM DTT, 1 mM magnesium acetate, 100 µM ATP, 4 mM creatine phosphate, 20 ng/µL creatine kinase, 50 mM potassium glutamate, 150 mM TMAC, 5% by mass PEG8000, and 10% by volume DMSO), and the reaction liquid was incubated at 42°C for 30 minutes and at 65°C for 2 minutes to ligate the plasmid and each OriC cassette. Using the same method as in Example 1, the target circular DNA was amplified by the RCR amplification reaction using the ligated DNA as the template.

**[Table 11]**

| Primer name | Sequence | SEQ ID NO |
|---|---|---|
| pBeloBAC-11_FW | ctatgcggcatcagagcag | 23 |
| pBeloBAC-11_RV | gttaagccagccccgacac | 24 |
| OriC1.0_FW | atggtgcactctcagtacaatctgctctgatgccgcatagtatgttgtaactaaa gatctactgtggataactctg | 25 |
| Ori C1.0_RV | cgcgtcagcgggtgttggcgggtgtcggggctggcttaacagtatgttgtaacta aagtattaaaaagaagatctatttatttag | 26 |
| OriG_FW_2 (OriC2.0_FW/OriCsg _FW/OriC5.6AT_FW) | cgcgtcagcgggtgttggcgggtgtcggggctggcttaacgagctgttgacagag ggtcattttc | 27 |
| OriC_RV_2 (OriC2.0_RV/OriCsg _RV/Ori C5. GAT_RV) | atggtgcactctcagtacaatctgctctgatgccgcatagtaaaaaaacagcctc cgaaaggag | 28 |

### [Example 4] Escherichia coli cloning of circular DNA incorporating OriC cassette

### [Example 4-1]

The circular DNAs obtained in Examples 1 and 2 were introduced into an Escherichia coli NEB stable strain (manufactured by NEB) by a chemical method. Then, a portion of the bacteria was cultured at 30°C for 24 hours in an LB agar medium containing 33 µg/mL kanamycin. A plurality of Escherichia coli colonies were selected from the agar plate medium, and each of the transformed Escherichia coli was subjected to shaking culture at 30°C for 24 hours in 5 mL of a liquid medium containing 33 µg/mL kanamycin. As the liquid medium, an LB liquid medium was used. A commercially available DNA extraction kit (product name "QIAprep Spin Miniprep Kit", manufactured by QIAGEN) was used for plasmid extraction. A portion of the plasmid DNA solution eluted with 50 µL of 10 mM Tris-Cl (pH 8.5) solution was treated with the restriction enzyme XbaI, and the concentration was measured with a fluorometer (product name "Quantus (registered trademark) Fluorometer", manufactured by Promega Corporation).

### [Example 4-2]

The circular DNAs obtained in Example 3 were introduced into an Escherichia coli DH5α strain (manufactured by Takara Bio Inc.) by a chemical method. Then, a portion of the bacteria was cultured at 37°C for 16 hours in an LB agar medium containing 20 µg/mL chloramphenicol. A plurality of Escherichia coli colonies were selected from the agar plate medium, and each of the transformed Escherichia coli was subjected to shaking culture at 37°C for 16 hours in 5 mL of a liquid medium containing 20 µg/mL chloramphenicol. As the liquid medium, an LB liquid medium was used. A commercially available DNA extraction kit (product name "QIAprep Spin Miniprep Kit", manufactured by QIAGEN) was used for plasmid extraction. A portion of the plasmid DNA solution eluted with 50 µL of 10 mM Tris-Cl (pH 8.5) solution was treated with the restriction enzyme XbaI, and the concentration was measured with a fluorometer (product name "Quantus (registered trademark) Fluorometer", manufactured by Promega Corporation).

### [Example 5]

RCR amplification reaction solutions were prepared by mixing 1 µL (200 pg/µL) of each of the solutions of the plasmids (6 types) obtained by incorporating three OriC cassettes (OriC1.0, OriC2.0, and OriCsg) into the two p15A plasmids and the plasmids (2 types) obtained by incorporating two OriC cassettes (OriC2.0 and OriCsg) into the pUC plasmid, which were obtained in Example 4, with 99 µL of the reaction mixture having the composition shown in Table 9, and the RCR amplification reaction solutions were incubated at 33°C for 10 hours to perform the RCR amplification reaction, and then cooled to 4°C.

Then, a portion of the reaction liquid was subjected to agarose gel electrophoresis together with a marker, and the separated bands were stained with dsGreen.

In addition, a portion of the reaction liquid was treated with the restriction enzyme XbaI, and the DNA concentration was measured with Quantus-PicoGreen system (Promega Corporation).

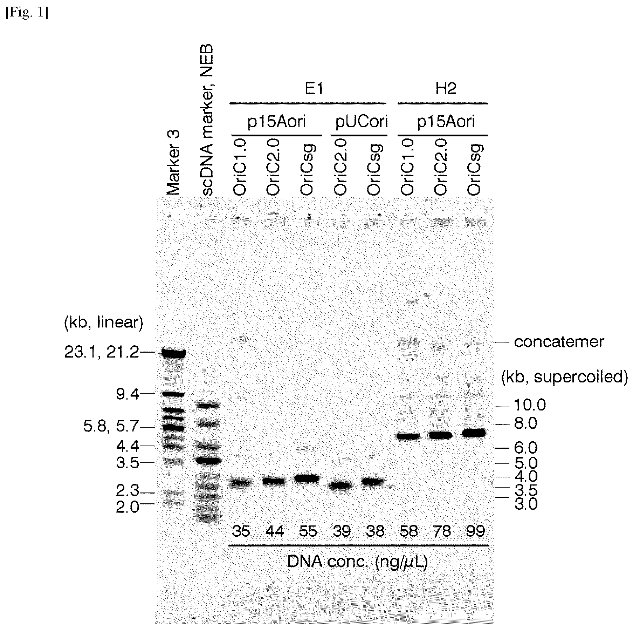

The results are shown in Fig. 1. The concentrations of the amplified DNAs were higher when the p15A plasmids and OriCsg were combined compared to the cases of using other OriC cassettes. No significant increase in the concentration of the amplified DNA was observed when the pUC plasmid and OriCsg were combined compared to the cases of using other OriC cassettes.

### [Example 6]

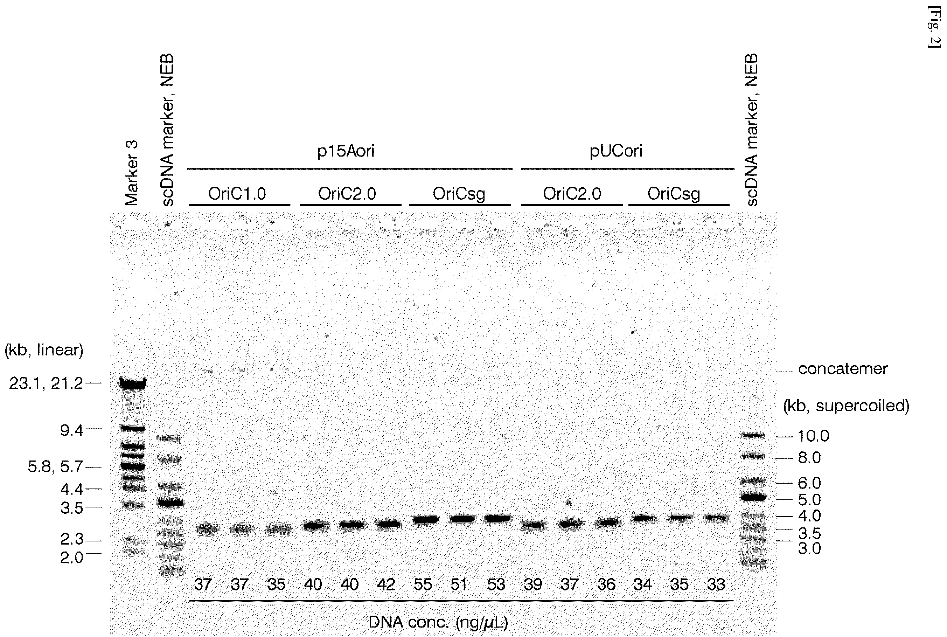

The samples of the five plasmids having the E1 sequence among the eight plasmids used in Example 5 were each subjected to the same experiment as in Example 5 three times. As shown in Fig. 2, the same results as in Example 5 were obtained, and the concentrations of the amplified DNAs were higher when the p15A plasmids and OriCsg were combined compared to the cases of using other OriC cassettes. Furthermore, as in Example 5, no significant increase in the concentration of the amplified DNA was observed when the pUC plasmid and OriCsg were combined compared to the cases of using other OriC cassettes. The mean values of the three experiments are shown in Table 12.

**[Table 12]**

| E1- | DNA conc. (mean ± SE, ng/µL) |
|---|---|
| p15Aori-OriC1.0 | 36.3 ± 0.7 |
| p15Aori-OriC2.0 | 40. 7 ± 0.7 |
| p15Aori-OriCsg | 53.0 ± 1.2 |
| pUCori-OriC2.0 | 37. 3 ± 0.9 |
| pUCori-OriCsg | 34.0 ± 0.6 |

### [Example 7]

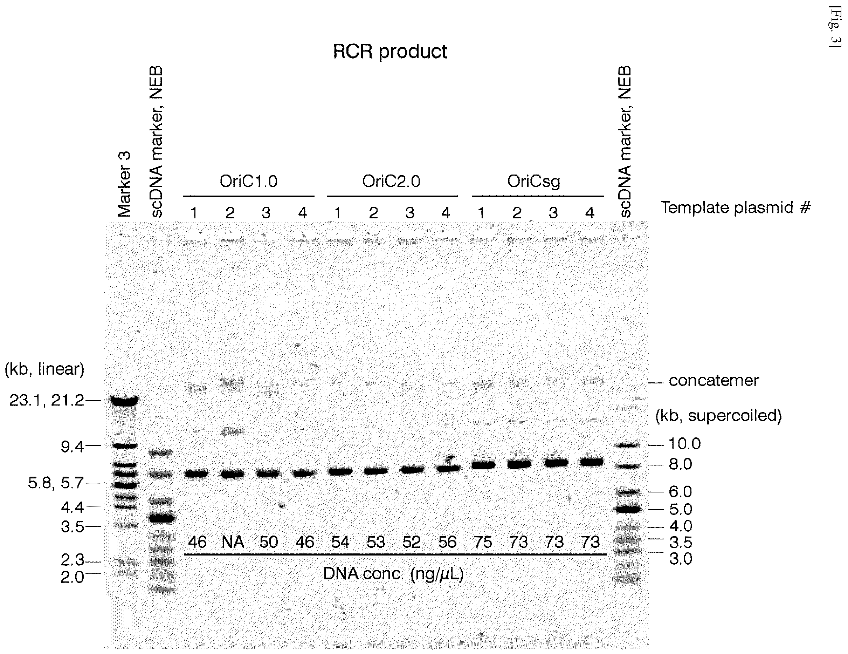

The samples of the three plasmids obtained in Example 4 which were pBeloBAC incorporating each of three OriC cassettes (OriC1.0, OriC2.0, and OriCsg) were each subjected to the same experiment as in Example 5 four times. As shown in Fig. 3, even when the pBeloBAC plasmid was used, the concentrations of the amplified DNAs were higher when the plasmid was combined with OriCsg compared to the cases of using other OriC cassettes, as in the case of using the p15A plasmids. The mean values of the experiments are shown in Table 13 (for the experiments in which the OriC1.0 cassette was used, the mean value was calculated from the results of three experiments).

**[Table 13]**

| pBeloBAC-11+ | DNA conc. (mean ± SE, ng/µL) |
|---|---|
| OriC1.0 | 47.3 ± 1.3, n=3 |
| OriC2.0 | 53.8 ± 0. 9, n=4 |
| OriCsg | 73.5 ± 0.5, n=4 |

### [Example 8]

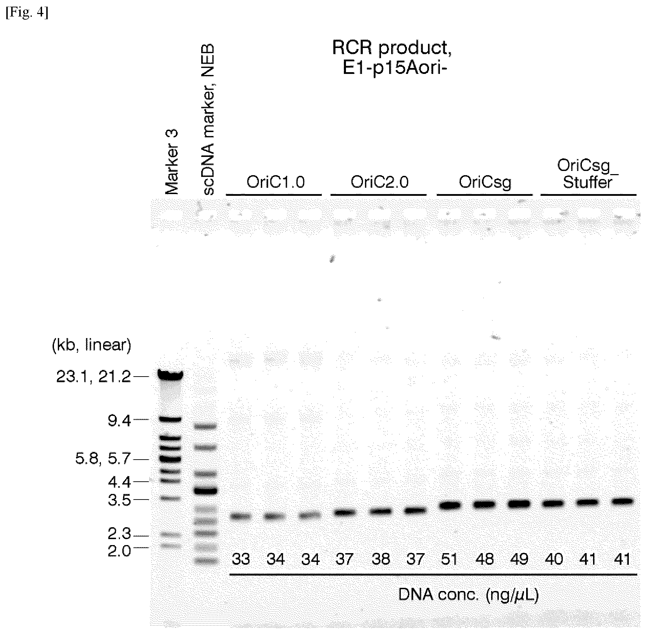

The samples of the four plasmids obtained in Example 4 which were E1-p15A plasmids incorporating each of four OriC cassettes (OriC1.0, OriC2.0, OriCsg, and OriC_Stuffer) were each subjected to the same experiment as in Example 5 three times. As shown in Fig. 4, even when the pBeloBAC plasmid was used, the concentrations of the amplified DNAs were higher when the plasmid was combined with OriCsg compared to the cases of using other OriC cassettes, as in the cases of using the p15A plasmids. On the other hand, when the MuSGS sequence in OriCsg was changed to the Stuffer sequence, the concentrations of the amplified DNAs were lower compared to the cases of using OriCsg. The mean values of the experiments are shown in Table 14.

**[Table 14]**

| E1-p15Aori- | DNA conc. (mean ± SE, ng/µL) |
|---|---|
| OriC1.0 | 33. 7 ± 0.3 |
| OriC2. 0 | 37.3 ± 0.3 |
| OriCsg | 49. 3 ± 0.7 |
| OriCsg_Stuffer | 40.7 ± 0.3 |

### [Example 9]

### [Example 9-1]

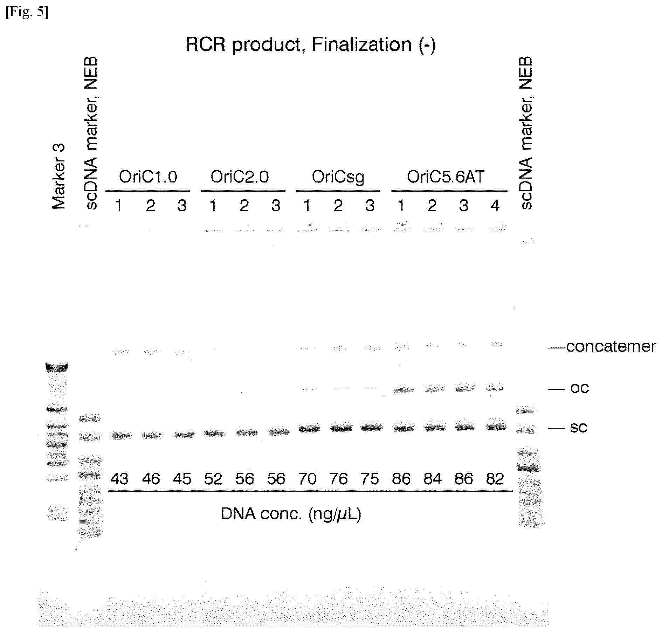

The samples of the four plasmids obtained in Example 4 which were pBeloBAC incorporating each of four OriC cassettes (OriC1.0, OriC2.0, OriCsg, and OriC5.6AT) were each subjected to the same experiment as in Example 5 three times. As shown in Fig. 5, when the pBeloBAC plasmid was used, the concentrations of the amplified DNAs were highest in the order of OriC5.6AT > OriCsg > OriC2.0 > OriC1.0. When the OriC5.6AT cassette was used, the amounts of the open circular DNA (OC) after the amplification were larger compared to the cases of using other OriC cassettes.

### [Example 9-2]

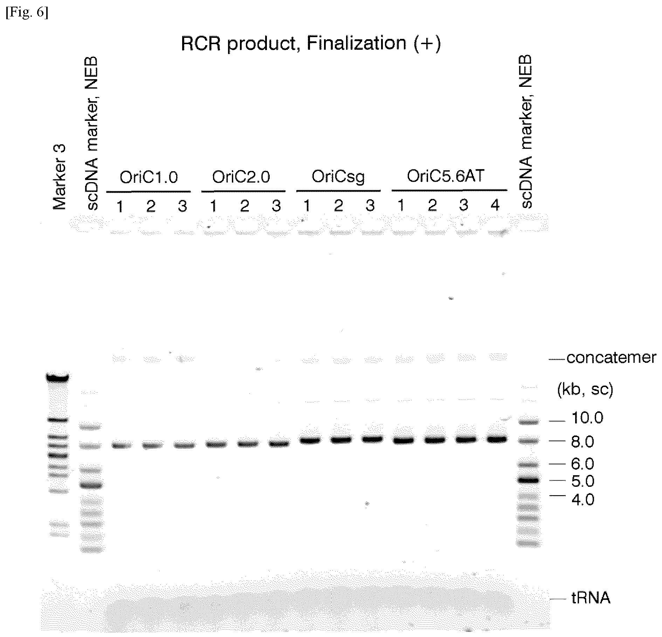

After the circular DNAs were amplified in the same manner as in Example 9-1, a portion (1 µL) of the RCR amplification reaction solution was diluted 1:5 with the RCR reaction buffer shown in Table 9. The diluted product was incubated at 30°C for 30 minutes and allowed to cool on ice (finalization treatment). Then, as in Example 9-1, a portion of each reaction liquid was subjected to agarose gel electrophoresis together with a marker, and the separated bands were stained with dsGreen. The results are shown in Fig. 6. Compared to Example 9-1, the amount of OC when the OriC5.6AT cassette was used was reduced.

### [Example 10]

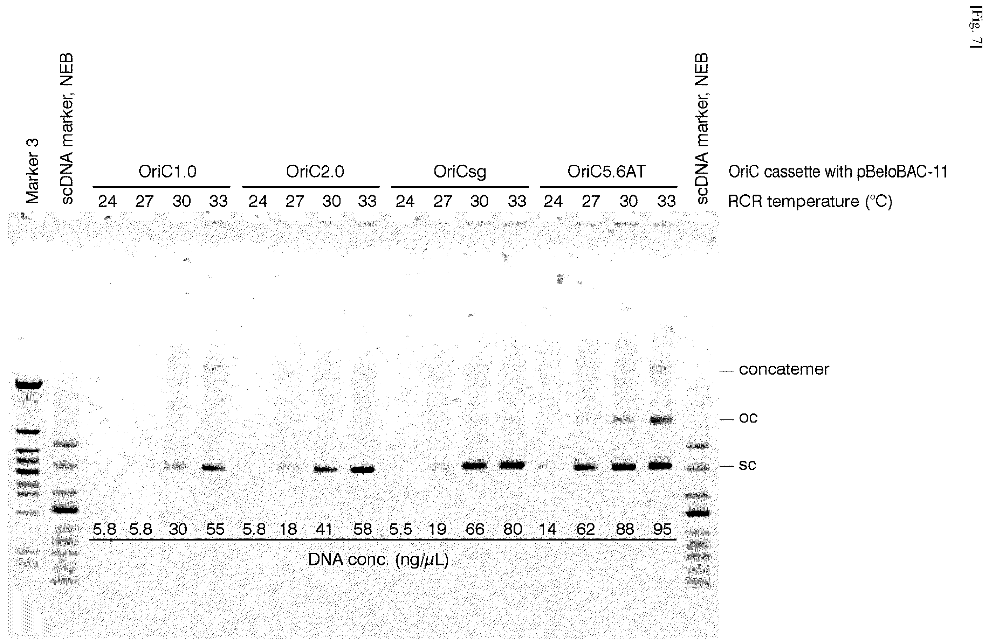

The same experiment as in Example 5 was performed except that the four plasmids used in Example 9 were used, and the RCR amplification reaction conditions were adjusted to a temperature of 24°C, 27°C, 30°C, or 33°C and reaction time of 10 hours. The results are shown in Fig. 7. As shown in Fig. 7, the circular DNA including OriC1.0 as the OriC cassette was hardly amplified at the temperatures of 27°C or lower. A small amount of amplification was observed at 27°C with the circular DNA including OriC2.0 or OriCsg. Amplification of the circular DNA including OriC5.6AT was observed even at 24°C, and thus the circular DNA was capable of being amplified at a lower temperature compared to the cases of using other OriC cassettes.

### [Example 11]

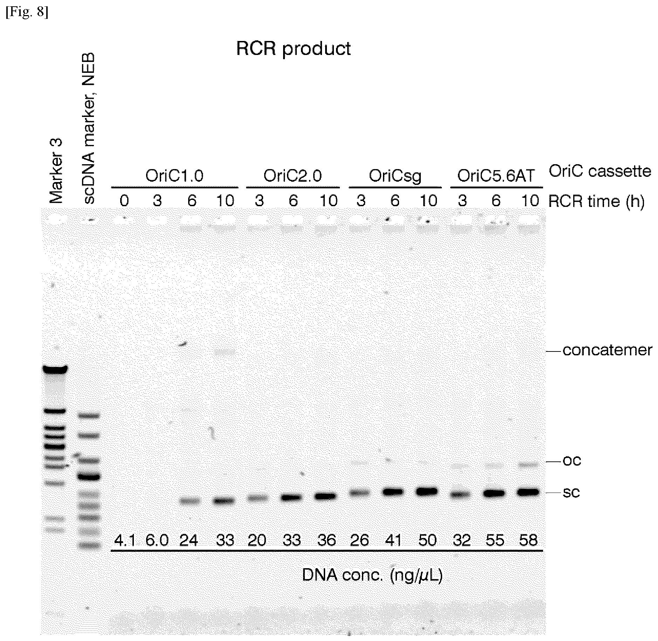

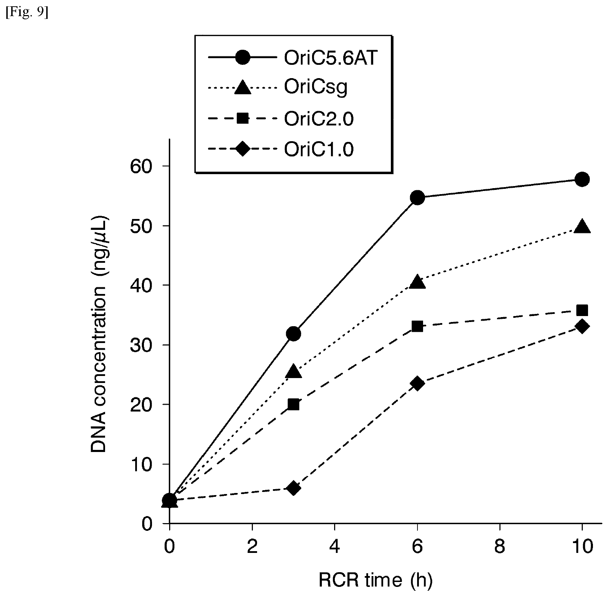

The same experiment as in Example 5 was performed except that the four plasmids obtained in Example 4 which were the E1-p15A plasmids incorporating each of four OriC cassettes (OriC1.0, OriC2.0, OriCsg, and OriC5.6AT) were used, and the RCR amplification reaction conditions were adjusted to a temperature of 33°C and reaction time of 3 hours, 6 hours, or 10 hours. The results are shown in Fig. 8. The changes in DNA concentration with reaction time are shown in the graph in Fig. 9 and in Table 15.

**[Table 15]**

| Time (h) | OriC5.6AT | OriCsg | OriC2.0 | OriC1.0 |
|---|---|---|---|---|
| 0 | 4.06 | 4.06 | 4.06 | 4.06 |
| 3 | 32 | 26 | 20 | 6 |
| 6 | 55 | 41 | 33 | 24 |
| 10 | 58 | 50 | 36 | 33 |

As shown in Figs. 8 and 9, the amount of DNA amplification was greatest when OriC5.6AT was used as the OriC cassette, and the amplification rate was also faster compared to the cases of using other cassettes. On the other hand, an increase in OC was observed as the amplification reaction time increased.

### [Example 12]

Some fragments of a commercially available pACYC184_DNA plasmid (Nippon Gene Co., Ltd.) having the replication initiation region derived from p15A plasmid and the fragments of the OriC cassettes (OriC2.0 and OriCsg) from Preparation Examples 2 and 3 were prepared by PCR using the primers listed in Table 16.

**[Table 16]**

| Primer name | Sequence | SEQ ID NO |
|---|---|---|
| pACYC184_FW | cagcagaatatgtgatacaggatatattcc | 58 |
| pACYC184_RV | acgcaccggtgcagccttttttc | 59 |
| OriC_pACYC184_FW | tgcttcatgtggcaggagaaaaaaggctgcaccggtgcgttaaaaaaacag cctccgaaaggag | 60 |
| OriC_pACYC184_RV | gcgaggaagcggaatatatcctgtatcacatattctgctggagctgttgac agagggtcattttc | 61 |

The fragments were ligated using the homologous recombination reaction in the same manner as in Example 3, and then the RCR amplification reaction was performed using the ligated DNA as the template to amplify the circular DNA in which OriC2.0 or OriCsg was incorporated into the pACYC184 plasmid. Subsequently, Escherichia coli cloning was performed by the same method as in Example 4-2.

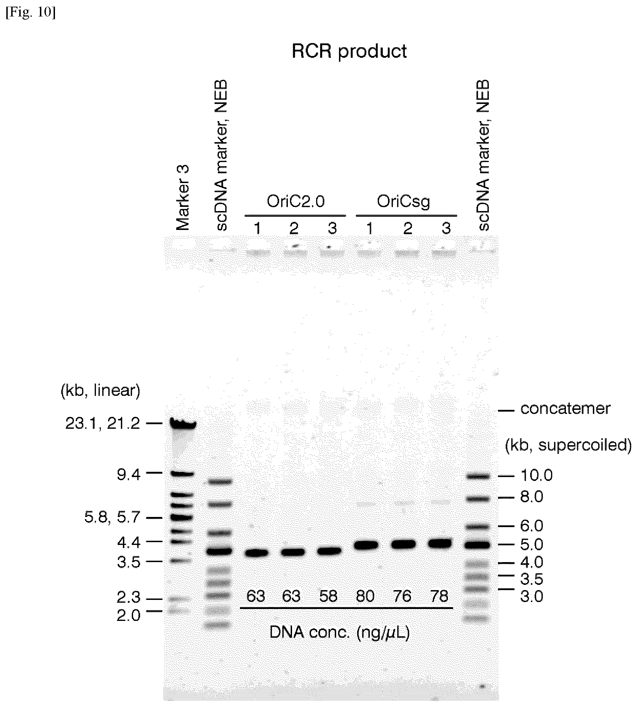

The RCR amplification reaction, agarose gel electrophoresis, and DNA concentration measurement were performed in the same manner as in Example 5 using 200 pg of each of the two obtained plasmids (plasmid having OriC2.0: 4671 bp and plasmid having OriCsg: 4827 bp) and 100 µL of the RCR reaction mixture. For RCR amplification, the reaction mixture with the composition shown in Table 9 was used after being diluted as appropriate. During the reaction, 0.05% Tween 20 (Wako Pure Chemical Industries, Ltd.) was added. The results are shown in Fig. 10. The mean values of the three experiments are shown in Table 17.

**[Table 17]**

| pACYC184+ | DNA conc. [mean ± SE (ng/µL)] |
|---|---|
| OriC2.0 | 61.3 ± 1.4 |
| OriCsg | 78.0 ± 0.9 |

It was confirmed that, even when the p15A plasmid different from those in Example 5 and the like was used, the concentrations of the amplified DNA were higher when the plasmid was combined with OriCsg compared to the case of combining the plasmid with OriC2.0.

### [Example 13]

Linear DNA without the Escherichia coli ori sequence was prepared from the pACYC184 plasmid used in Example 12. The fragments of each of the OriC cassettes and some fragments of the pACYC184 plasmid from Example 12 were each prepared by PCR using the primers listed in Table 18.

**[Table 18]**

| Primer name | Sequence | SEQ ID NO |
|---|---|---|
| pACYC184_deIori_FW | cttcaaatgtagcacctgaagtc | 62 |
| pACYG184_RV | acgcaccggtgcagccttttttc | 63 |
| OriC_pACYC1B4_FW | tgcttcatgtggcaggagaaaaaaggctgcaccggtgcgttaaaaaa acagcctccgaaaggag | 64 |
| OriC_pACYC184_deIori_RV | ttatatcgtatggggctgacttcaggtgctacatttgaaggagctgt tgacagagggtcattttc | 65 |

The prepared linear DNA and the fragments of the OriC cassettes (OriC2.0 and OriCsg) were ligated using the homologous recombination reaction in the same manner as in Example 12, and then the RCR amplification reaction was performed using the ligated DNA as the template to amplify the circular DNA in which OriC2.0 or OriCsg was incorporated into the pACYC184 plasmid without ori.

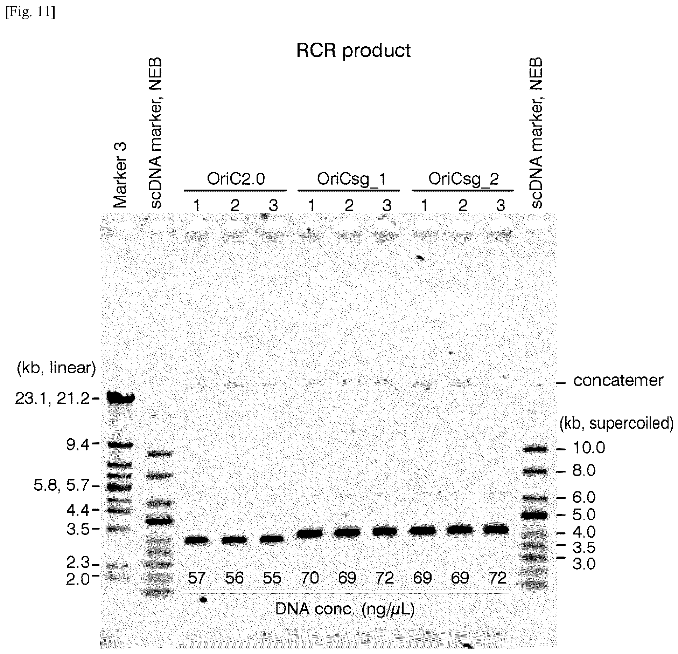

The RCR amplification reaction, agarose gel electrophoresis, and DNA concentration measurement were performed in the same manner as in Example 5 using 200 pg of the two obtained circular DNAs (circular DNA having OriC2.0: 3901 bp and circular DNA having OriCsg: 4057 bp) and 100 µL of the RCR reaction mixture. For RCR amplification, the same reaction mixture as that in Example 12 was used. The results are shown in Fig. 11. For OriCsg, the experiment was performed on two lots. The mean values of the three experiments are shown in Table 19.

**[Table 19]**

| pACYC184_de I or i | DNA conc. [mean ± SE (ng/µL) ] |
|---|---|
| OriC2.0 | 56. 0 ± 0.5 |
| OriCsg Iot 1 | 70. 3 ± 0.7 |
| OriCsg Iot 2 | 70.0 ± 0.8 |

It was confirmed that, even when the p15A plasmid not having the Escherichia coli ori was used, the concentrations of the amplified DNA were higher when the plasmid was combined with OriCsg compared to the case of combining the plasmid with OriC2.0.

### [Example 14]

In Example 14, the following method was used.

### <Amplification by RCR method>

Based on the previously reported methods (Non-Patent Documents 1 and 2), an amplification reaction was performed by the RCR method with slight modifications. 5 µL of an RCR reaction mixture containing 1× RCR buffer I, 1× RCR buffer II, 1× enzyme mix, and 3 ng/µL λ phage DNA was incubated at 33°C for 12 hours. The RCR reaction product was diluted with 1× RCR buffer and incubated at 30°C for 30 minutes. When the template was AT-rich circular DNA, AT-RCR was used in which an RCR reaction mixture obtained by adding 300 nM HU and 100 nM H-NS to a previously reported RCR reaction mixture was used to enhance the amplification. In an RCR amplification experiment conducted at a low temperature, 10 µL of a reaction mixture containing 1× RCR buffers I and II, 0.05% Tween 20, and 0.5× enzyme mix was used. The RCR reaction product was diluted with 1× RCR buffer and incubated at 30°C for 30 minutes. Some of the reaction product was mixed with a stop buffer (Non-Patent Document 2) and analyzed by agarose gel electrophoresis. Assembly of a plurality of DNAs was performed by the RA method (WO 2019/009361 A).

The agarose gel electrophoresis was performed as previously reported (Nara and Su'etsugu, Biotechniques, 2021, vol. 71, p.528-533.).

### <Construction of OriC mutants>

The construction of each mutant and circular DNA incorporating the mutant is described in detail below. PCR amplification was performed using KOD One polymerase (manufactured by TOYOBO CO., LTD.). The obtained fragments were subjected to gel extraction and purified by column purification (manufactured by Macherey-Nagel GmbH & Co. KG). The obtained oriC mutant fragments were incorporated into the pSV-β-galactosidase (pSVβ; manufactured by Promega Corporation) vector (7.2 kb) by the previously reported method (WO 2023/038145 A, Example 1) using Escherichia coli RecA and exonuclease III, and amplified by the RCR method. The obtained amplified product was produced by column purification (manufactured by Macherey-Nagel GmbH & Co. KG).

### <Real-time RCR>

Each reaction was carried out using 10 µL of a reaction mixture containing final concentrations of 1× RCR buffers I and II, 0.05% Tween 20, 300 nM YO-PRO-1 iodine (Ex/Em = 491/509 nm) (manufactured by Thermo Fisher Scientific Inc.), and 0.5× RE enzyme mix. After pre-incubating 9 µL of the reaction mixture at 20°C for 15 minutes, the mixture was placed on ice to prevent the start of the RCR reaction. Next, 1 µL of oriC DNA was added to the final concentration of 1 pM, and the RCR reaction was started at 30°C using a real-time system ("Thermal Cycler Dice Real time system", manufactured by Takara Bio Inc.). The amplified DNA was detected using a SYBR filter every 10 minutes. The doubling time of the amplified DNA in the RCR system was calculated from the slope of the logarithmic phase in the amplification curve. To standardize the experimental conditions for each oriC mutant, all the mutants were cloned into a pSVβ plasmid with a GC content of 52%.

### <Competitive amplification assay>

A 13-kb minichromosome including oriCwt which was used as a competitor was mixed at a 200 pM: 100 pM ratio with a 7-kb minichromosome including the oriC mutant. The DNA mixture was amplified by the RCR amplification method described above with a slight modification. Each RCR reaction was performed using 10 µL of a reaction mixture containing final concentrations of 1× RCR buffers I and II, 0.05% Tween 20, and 0.5× RE enzyme mix. The RCR reaction mixture was pre-incubated at 20°C for 15 minutes and mixed on ice with the DNA mixture to prevent the start of the RCR reaction. After the addition of the DNA mixture, amplification was carried out at 30°C for 12 hours by the RCR method. The RCR reaction product was diluted with 1× RCR buffer, incubated at 30°C for 30 minutes, and mixed with a stop buffer. Some of mixture was then mixed with the stop buffer and analyzed by agarose gel electrophoresis.

### [Example 14-1] Construction of circular DNA incorporating OriC mutant

### <Circular DNA incorporating OriCwt>

Wild-type oriCwt having the sequence listed in Table 20 was subjected to PCR amplification using the primer pair P1/P2 listed in Table 21.

**[Table 20]**

| OriCwt (underlined portion: minimal 245-bp oriC) | SEQ ID NO |
|---|---|
| 5' -gtcattttcacactataatgccgctatagtgagtcgtagtatgttgtacctaaagggatcctgggtatta aaaagaagatctatttatttagagatctgttctattgtgatctcttattaggatcgcactgccctgtggataac aaggatccggcttttaagatcaacaacctggaaaggatcattaactgtgaatgatcggtgatcctggaccgtat aagctgggatcagaatgaggggttatacacagctgaggaactgaagagcagttgttctttggataactaccggt tgatccaagcttcctgacagagttatccacagtagatcgcacgatctgtcagctcatttcctttaggtacaaca tactagaatatttgcctacagcctccttt -3' | 66 |

The resulting amplified product was introduced into a pSV-β-galactosidase vector (pSVβ) (6.8 kb, 53% GC) (manufactured by Promega Corporation) and amplified by the method described in Example 1 of WO 2023/038145 A to obtain a minichromosome having oriCwt (pSVβ-oriCwt).

### <Circular DNA incorporating oriC2.0AT, oriC3.0AT, oriC3.4AT, or oriC3.6AT>

Using the primer pairs listed in Table 21, minichromosomes having oriC2.0AT, oriC3.0AT, or oriC3.4AT were constructed. Specifically, among the primer pairs listed in Table 21, P1/P4 and P2/P3, P1/P6 and P2/P5, and P1/P8 and P2/P7 were used, respectively. The oriC region of the template pSVβ-oriCwt DNA was then divided into two and amplified by PCR using these two primer pairs separately. Thereafter, the two obtained fragments were assembled by overlap PCR using the P1/P2 primers and then introduced into the pSVβ vector using the method described in Example 1 of WO 2023/038145 A.

A minichromosome having oriC3.6AT was constructed using the following primer pairs. The oriC region of the template pSVβ-oriC3.0AT DNA was divided into two and amplified by PCR using P1/P10 and P2/P9 among the primer pairs listed in Table 21. Thereafter, the two obtained fragments were assembled by overlap PCR using the P1/P2 primers and then introduced into the pSVβ vector using the method described in Example 1 of WO 2023/038145 A.

### <Circular DNA incorporating oriC4.0AT, oriC5.4AT, or oriC5.6AT>

oriC4.0AT was constructed as follows. PCR amplification was performed using the primer pairs P1/P12 and P2/P7 listed in Table 21 and oriC3.6AT as the template, and the two obtained fragments were assembled by overlap PCR using P1/P2, and then introduced into the pSVβ vector using the method described in Example 1 of WO 2023/038145 A.

oriC5.4AT and oriC5.6AT were constructed using the primer pairs P1/P12 and P2/P11, and P1/P14 and P2/P13, respectively. These primer pairs were used in PCR amplification performed using oriC3.4AT and oriC3.6AT as the templates. The assembled fragments produced by the overlap PCR using P1/P2 was introduced into the pSVβ vector using the method described in Example 1 of WO 2023/038145 A, thus obtaining circular DNAs incorporating oriC5.4AT and oriC5.6AT.

### <Circular DNA incorporating oriC6.0AT>

oriC6.0AT was constructed as follows. The fragments obtained by using the primer pairs P1/P14, P12/P13, and P2/P11 listed in Table 21 and oriC4.0AT as the template were assembled by overlap PCR using P1/P2. The resulting assembly product was introduced into the pSVβ vector using the method described in Example 1 of WO 2023/038145 A.

A minichromosome including the oriC mutant, which were created and amplified using the method described in Example 1 of WO 2023/038145 A, were purified using a spin column (product name "QIAprep Spin Miniprep Kit", manufactured by Qiagen). The sequence of the oriC mutant was confirmed by Sanger sequencing.

### <Circular DNA incorporating oriCdueM9T, oriCdueM9A, oriCdueM13T, or oriCdueM13A>

A 7-kb minichromosome including a poly A or T sequence in the DUE-M or DUE-R region was constructed. oriCdueM9T, oriCdueM9A, oriCdueM13T, and oriCdueM13A were designed with the primer pairs P15/P16, P17/P18, P19/P20, and P21/P22 listed in Table 21, respectively. These primers were then used in subsequent inverse PCR amplification performed using pSVβ-oriCwt DNA as the template.

Then, 100 µL of competent Escherichia coli DH5α cells (cell name "DH5α high Champion", manufactured by SMOBIO Technology, Inc.) were directly transformed with 1 µL of the obtained fragments. The resulting transformants were selected on an LB plate containing 50 µg/mL carbenicillin at 30°C. These transformed cells were cultured at 30°C for 16 hours, and then minichromosome DNAs including oriCdueM9T, oriCdueM9A, oriCdueM13T, and oriCdueM13A were purified from the cells using a spin column (product name "QIAprep Spin Miniprep Kit", manufactured by Qiagen). The sequence of the oriC mutant was confirmed by Sanger sequencing.

**[Table 21]**

| Primer name | Sequence | SEQ ID NO |
|---|---|---|
| P1 | GATCTAGGTGAAGATCCTTTTTGATAATCTCATGACCAAAATCCCTTAACGTGAGTTTTCGTCAT TTTCACACTATAATG | 67 |
| P2 | TTTTTGTTTGCAAGCAGCAGATTACGCGCAGAAAAAAAGGATCTCAAGAAGATCCTTTGAAAAGG AGGCTGTAGGCAAATATTCT | 68 |
| P3 | GGTAAATATATTTATATGATTATATTTATTATATTAACTATATTTATTGCTATTATCGCACTGCC CTGTGGATAACAAGG | 69 |
| P4 | TTAATATAATAAATATAATCATATAAATATATTTACCAATTCCATCATTTTGAAACTTTAGGTAC AACATACTACGACTC | 70 |
| P5 | TTAGAGATATGTTCTATTGTGATTATTATTAGGATCGCACTGC | 71 |
| P6 | CCTAATAATAATCACAATAGAACATATCTCTAAATAAATAGATC | 72 |
| P7 | GTGAATGATCTGTGATCCTGGACTGTATAAGCTGTGATCAGAATG | 73 |
| P8 | ACAGCTTATACASTCCAGGATCACAGATCATTCACAGTTAAT | 74 |
| P9 | ATTTATATATATATTCTATTGTGATTATTATTAGGATCGCACTGC | 75 |
| P10 | CCTAATAATAATCACAATAGAATATATATATAAATAAATAGATC | 76 |
| P11 | AGGATCATTAACTGTGAATAATCTGTGATCCTGGACTGTA | 77 |
| P12 | TACAGTCCAGGATCACAGATTATTCACAGTTAATGATCCT | 78 |
| P13 | TTTATATATATATTATATTATTATTATTATTAGGATCGCACTGC | 79 |
| P14 | CCTAATAATAATAATAATATAATATATATATAAATAAATAGATC | 80 |
| P15 | TTTATTTAGTTTTTTTTTCTATTGTGATCTCTTATTAGGATCGCACTGCC | 81 |
| P16 | ACAATAGAAAAAAAAACTAAATAAATAGATCTTCTTTTTAATAGCCAGGA | 82 |
| P17 | TTTATTTAGAAAAAAAAACTATTGTGATCTCTTATTAGGATCGCACTGCC | 83 |
| P18 | ACAATAGTTTTTTTTTCTAAATAAATAGATCTTCTTTTTAATACCCAGGA | 84 |
| P19 | TATTGTTTTTTTTTTTTTTGATCGCACTGCCCTGTGGATAACAAGGATCC | 85 |
| P20 | GCGATCAAAAAAAAAAAAAAGAATAGAACAGATCTCTAAATAAATAGATC | 86 |
| P21 | TATTGTAAAAAAAAAAAAAGATCGCACTGCCCTGTGGATAACAAGGATCC | 87 |
| P22 | GCGATCTTTTTTTTTTTTTACAATAGAACAGATCTCTAAATAAATAGATC | 88 |

### [Example 14-2] RCR amplification by OriC mutants with mutations in DUE

Fig. 12A is a schematic diagram of an amplification cycle by the RCR method. The amplification process starts from the replication initiation sequence (oriC) and exponentially amplifies the circular DNA through bidirectional amplification, resulting in supercoiled DNA. An initiation step proceeds only when the double-stranded DNA at the oriC is cleaved.

Fig. 12B is a schematic diagram of the initiation process at the oriC. The oriC includes an AT-rich double-strand cleavage region (DUE) and a DnaA assembly region (DOR), the DnaA being an initiator protein. ATP-bound DnaA (ATP-DnaA) binds to the DOR and forms a homomultimeric complex, which cleaves the AT-rich DUE and allows entry of DnaB helicase. The dark arrows (R1, R5M, R2, and R4) indicate the DnaA-ADP binding sites. The light arrows (τ2, l1, l2, C3, C2, l3, and C1) indicate the DnaA-ATP-binding sites.

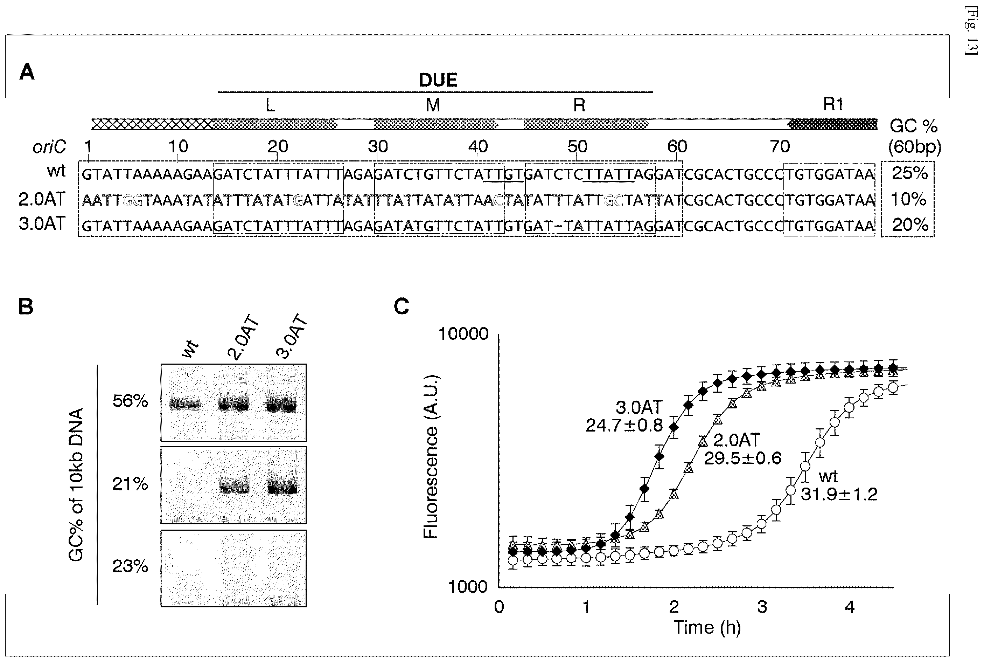

The effects of OriC mutants having mutations in the DUE on the RCR amplification efficiency were examined. Fig. 13A shows the schematic diagram of the oriC2.0AT and oriC3.0AT sequences created in Example 14-1. DUE L, M, and R represent repetitive 13-mer sequences. R1 represents a DnaA-box R1 sequence. The dotted box shows the regions (1 to 60 bp) used to calculate the GC contents which are shown to the right of the sequences. The sequences in Fig. 13A are shown in Table 22. In Table 22, the underlines in the wild type indicate the DnaA-ssDUE binding sequences (TT[A/G]T(T)), the letters enclosed by boxes are the bases different from those in the wild type sequence, and the "-" represents a base deletion. Each base number was assigned starting from the region 70 bp upstream from the base immediately before the base of the R1 DnaA-box.

**[Table 22]**

| Cassette name | Sequence | GC content (60 bp) | SEQ ID NO |
|---|---|---|---|
| OriCwt | GTATTAAAAAGAAGATCTATTTATTTAGAGATCTGTTCTATTGTGATCTCTT ATTAGGATCGCACTGCCCTGTGGATAA | 25% | 89 |
| OriC2.OAT | AATTGGTAAATATATTTATATGATTATATTTATTATATTAACTATATTTA TTGCTATTATCGCACTGCCCTGTGGATAA | 10% | 90 |
| OriC3.OAT | GTATTAAAAAGAAGATCTATTTATTTAGAGATATGTTCTATTGTGAT- TATTATTAGGATCGCACTGCCCTGTGGATAA | 20% | 91 |

10-kb fragments with a GC content of 56%, 21%, or 23% were assembled with oriC2.0AT (2.0 AT), oriC3.0AT (3.0 AT), or oriCwt (wt) and amplified using the RCR method. The resulting RCR product was subjected to agarose gel (1%) electrophoresis in 0.5× TBE and stained with SYBR green I. The results are shown in Fig. 13B. As shown in Fig. 13B, when the OriC2.0AT and OriC3.0AT cassettes were used, the amplification efficiency of circular DNA with a low GC content (that is, circular DNA with high AT content), which cannot be amplified with the wild-type oriC, was increased compared to the case of using the wild type. It was presumed that, while the 10-kb fragment with a GC content of 21% was amplified, the 10-kb fragment with a GC content of 23% was not amplified, since the 10-kb fragment with a GC content of 23% contained a region where the double strand derived from the DUE of oriC2.0AT was easily cleaved, and thus the DNA structure (negative supercoil) necessary for the initiation of replication was less likely to be formed.

Real-time RCR of oriCwt, oriC2.0AT, and oriC3.0AT was performed. The amplification of 7-kb circular DNA (pSVβ, 52% GC) including each oriC was started at 30°C at a DNA concentration of 1 pM in the presence of a YO-PRO-I intercalator. The results are shown in Fig. 13C. The numerical values accompanying each graph are the doubling time and standard deviation. As shown in Fig. 13C, for oriC2.0AT and oriC3.0AT, an enhanced rise time of the amplification curve was observed, suggesting an improvement in amplification efficiency in the early stage of the PCR amplification.

Amplification rates were calculated using the doubling time calculated from the slope of the logarithmic plots of the amplification shown in Fig. 13C. Real-time RCR analysis showed that the amplification rates of oriC2.0AT (29.5 minutes) and oriC3.0AT (24.7 minutes) increased by 1.1-fold and 1.3-fold, respectively, compared to oriCwt (31.9 minutes). Despite the low GC content in the DUE, the amplification rate of oriC2.0AT (10%) was slower than that of oriC3.0AT (20%) (high GC content), suggesting that, in addition to the reduced GC content in the DUE region, sequence specificity enhances the amplification rate.

In the DUE of oriC2.0AT, three nine-base repeat sequences of 5'-TATATTTAT-3' were observed, spaced 5 and 9 bases apart (Fig. 13A).

The mutations in the DUE of oriC3.0AT are two substitutions of C by A and a single-base deletion mutation (Fig. 13A). These mutations resulted in a 5% reduction in the GC content compared to oriCwt. The number of KAK sequences (the number of consecutive KAK motifs) in the DUE-R increased from 3 to 5.

These DUE mutations also reduced the GC content and increased the number of TAT sequence motifs (for example, TTATT) which resembled the DnaA-ssDUE binding sequence TT[A/G]T(T).

### [Example 14-3] RCR amplification 1 by OriC mutants with mutations in DUE and DOR

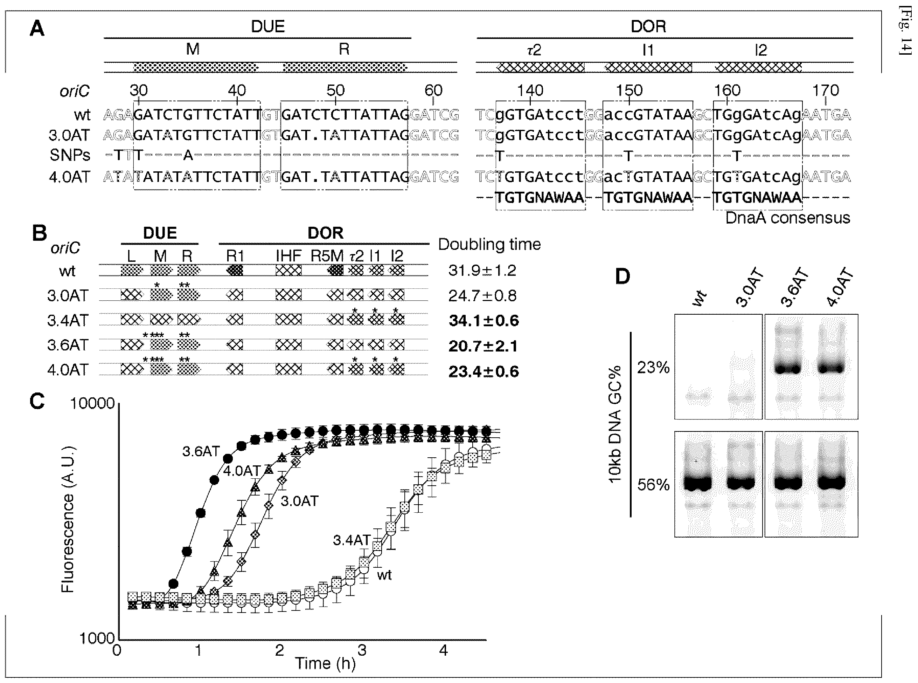

The effects of OriC mutants having mutations in the DUE and DOR on the RCR amplification efficiency were examined. Fig. 14A shows the schematic diagram of the oriC3.0AT and oriC4.0AT sequences created in Example 14-1. In Fig. 14A, the mutations that oriC4.0AT has compared to oriC3.0AT are shown on the lines of SNPs. In the sequences, "-" represents a base deletion, and the lowercase letters represent sequences that do not match the DnaA consensus sequence in the DOR region. As in Fig. 13, each base number was assigned starting from the region 70 bp upstream from the base immediately before the base of the R1 DnaA-box.

The three sequences in Fig. 14A are shown in Tables 23 and 24. The letters enclosed by boxes represent bases different from those of the wild-type sequence. The lowercase letters represent sequences that do not match the DnaA consensus sequence in the DOR region. In Table 23, the underlined regions represent the M and R elements of the DUE from the 5' side, and "." indicates a base deletion. In Table 24, the underlined regions represent the τ2, I1, and I2 elements of the DOR from the 5' side.

**[Table 23]**

| Cassette name | Sequences of DUE (M and R) portions | SEQ ID NO |
|---|---|---|
| Ori Cwt | AGAGATCTGTTCTATTGTGATCTCTTATTAGGATCG | 92 |
| OriC3.OAT | AGAGATATGTTCTATTGTGAT. TATTATTAGGATCG | 93 |
| OriC4.OAT | ATATATATATTCTATTGTGAT. TATTATTAGGATCG | 94 |

**[Table 24]**

| Cassette name | Sequences of DOR (τ2, I1, and I2) portions | SEQ ID NO |
|---|---|---|
| OriCwt | TCgGTGAtcctGGaccGTATAAGCTGgGAcAgAATGA | 95 |
| OriC3.OAT | TCgGTGAtcctGGaccGTATAAGCTGgGAtcAgAATGA | 96 |
| OriC4.OAT | TCTGTGAtcctGGacTGTATAAGCTGTGAtcAgAATGA | 97 |

Fig. 14B shows a schematic diagram of mutations on oriC3.0AT, oriC3.4AT, oriC3.6AT, and oriC4.0AT. The asterisks indicate the positions of the mutated bases that differ from the wild type. oriC3.4AT is a cassette that only has the mutations in the DOR of oriC4.0AT. oriC3.6AT is a cassette that only has the mutations in the DUE of oriC4.0AT. Using these mutant oriC cassettes, circular DNA amplification was performed in the same manner as in the real-time RCR method in Example 14-2, and the calculated doubling time (minutes) of the DNA amplification and standard deviations are shown at the rightmost part of Fig. 14B.

Fig. 14C shows the results of real-time RCR performed in the same manner as in Example 14-2 using oriCwt, oriC3.0AT, oriC3.4AT, oriC3.6AT, and oriC4.0AT.

As shown in Fig. 14A, compared to oriC3.0AT, oriC4.0AT has three mutations in the DOR region: G137T in τ2, C150T in I1, and G161T in I2. As shown in Figs. 14B and C, real-time RCR analysis revealed that the amplification rate of oriC4.0AT (23.4 minutes) was 1.4 times and 1.05 times higher than those of oriCwt (31.9 minutes) and oriC3.0AT (24.7 minutes), respectively. oriC3.6AT, which was the cassette only having the mutations in the DUE of oriC4.0AT, had an even higher (1.1 times) amplification rate (20.7 minutes) and a faster rise in the amplification curve compared to oriC4.0AT. In contrast, oriC3.4AT, which was the cassette only having the mutations in the DOR of oriC4.0AT, had an amplification rate (34.1 minutes) that was 0.7 times lower than that of oriC4.0AT and a rise in the amplification curve similar to that of oriCwt. These results suggested that the mutations in the DUE significantly improve the amplification rate. Compared to the wild-type oriC, the oriC cassettes that showed increased amplification rates had consecutive KAK sequences in the R element of the DUE region, and the oriC cassettes with particularly high amplification rates further included repetitive AT sequences on the left side (5' side) of the M element of the DUE region.

Fig. 14D shows the results of amplifying circular DNAs with different GC contents as in Example 14-2, using oriCwt, oriC3.0AT, oriC3.6AT, and oriC4.0AT. The circular DNA was obtained by assembling 10-kb DNA fragments with GC contents of 23% and 56% with each oriC cassette. The resulting DNA was amplified by the RCR method, subjected to agarose gel (1%) electrophoresis in 0.5x TBE, and then stained with SYBR green I.

As shown in Fig. 14D, when oriC3.6AT and oriC4.0AT were used, it was possible to amplify the circular DNA with a GC content of 23%, which was not amplified when oriC3.0AT was used in Example 14-2. As shown in Fig. 14A, compared with oriC3.0AT, oriC3.6AT and oriC4.0AT include repetitive AT sequences on the left side of the M element in the DUE region and have consecutive KAK sequences in the R element in the DUE region. It is thought that having such sequences allowed the amplification of DNA with a high AT content to become possible.

### [Example 14-4] RCR amplification 2 by OriC mutants with mutations in DUE and DOR

It was confirmed whether the DNA amplification rate in the RCR method was changed by further reducing the GC content in DUE and increasing the number of KAK sequences compared with Example 14-3. In addition, an additional mutation was introduced to R5M of DOR, which is particularly important for initiating DnaA homooligomer formation, and it was confirmed whether the DNA amplification rate in the RCR method was changed.

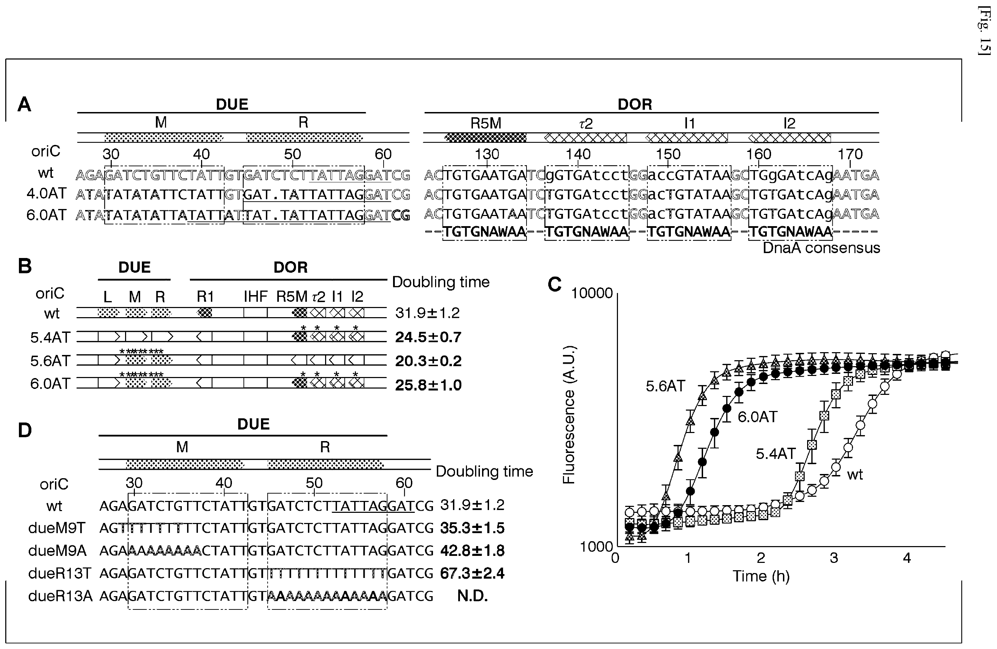

The effects of OriC mutants having mutations in the DUE and DOR on the RCR amplification efficiency were examined. Fig. 15A shows the schematic diagram of the oriC4.0AT and oriC6.0AT sequences created in Example 14-1. In the sequences in Fig. 15A, "-" represents a base deletion, and the lowercase letters represent sequences that do not match the DnaA consensus sequence in the DOR region. The underlines indicate consecutive KAK sequences. As in Fig. 13, each base number was assigned starting from the region 70 bp upstream from the base immediately before the base of the R1 DnaA-box.

The three sequences in Fig. 15A are shown in Tables 25 and 26. The letters enclosed by boxes represent bases different from those of the wild-type sequence, the lowercase letters represent sequences that do not match the DnaA consensus sequence in the DOR region, and the shaded letters represent consecutive KAK sequences. In Table 25, the underlined regions represent the M and R elements of the DUE from the 5' side, and "." indicates a base deletion. In Table 26, the underlined regions represent the R5M, τ2, I1, and I2 elements of the DOR from the 5' side. As shown in Table 25, oriC4.0AT and oriC6.0AT include five and seven KAK motifs in the DUE-R, respectively, and the GC contents are reduced to 10%. As shown in Table 26, the R5M sequence of the DOR region has a sequence that matches the consensus sequence of DnaA (Fig. 15A).

**[Table 25]**

| Cassette name | Sequences of DUE (M and R) portions | SEQ ID NO |
|---|---|---|
| OriCwt | AGAGATCTGTTCTATTGTGATCTCTTATTAGGATCG | 98 |
| OriC4,OAT | ATATATATATTCTATTGTGAT.TATTATTAGGATCG | 99 |
| OriC6.OAT | ATATATATATTATATTATTAT. TATTATTAGGATCG | 100 |

**[Table 26]**

| Cassette name | Sequences of DOR (R5M, τ2, I1, and I2) portions | SEQ ID NO |
|---|---|---|
| OriCwt | ACTGTGAATGATCgGTGAtcctGGaccGTATAAGCTGgGAtcAgAATGA | 101 |
| OriC4.OAT | ACTGTGAATGATCTGTGAtcctGGacTGTATAAGCTGTGAtcAgAATGA | 102 |
| OriC6.OAT | ACTGTGAATAATCTGTGAtcctGGacTGTATAAGCTGTGAtcAgAATGA | 103 |

Fig. 15B shows a schematic diagram of mutations on oriC5.4AT, oriC5.6AT, and oriC6.0AT. The asterisks indicate the positions of the mutated bases that differ from the wild type. oriC5.4AT is a cassette that has the same mutations as those in the DOR of oriC6.0AT. oriC5.6AT is a cassette having the sequence shown in Table 7, and particularly has the same mutations as those in the DUE (M and R) of oriC6.0AT. Using these mutant oriC cassettes, circular DNA amplification was performed in the same manner as in the real-time RCR method in Example 14-2, and the calculated doubling time (minutes) of the DNA amplification and standard deviations are shown at the rightmost part of Fig. 15B.

Fig. 15C shows the results of real-time RCR performed in the same manner as in Example 14-2 using oriCwt, oriC5.4AT, oriC5.6AT, and oriC6.0AT.

As shown in Figs. 15B and C, the doubling time of oriC6.0AT (25.8 minutes) was slower than that of oriC4.0AT (23.4 minutes), decreasing by 0.9-fold. On the other hand, the DNA amplification rate was increased and was slightly faster than that of oriC3.6AT (20.7 minutes; Figs. 14B and C) when oriC5.6AT (20.3 minutes) having only the mutations in DUE of oriC6.0AT was used. The number of KAK sequences in DUE-R is seven in oriC5.6AT and five in oriC3.6AT.

On the other hand, the DNA amplification rate was also increased by 1.05-fold compared to that of oriC6.0AT when oriC5.4AT (24.5 minutes) having only the mutations in DOR of oriC6.0AT was used. When oriC5.4AT was used, the DNA amplification rate in the RCR method was higher (1.3 times) compared to that of oriCwt. However, the rise of the amplification curve was slightly faster than that of oriCwt. The replication was also terminated earlier than with oriCwt.

The effects of an increase in A/T sequences in the DUE-MR region on the RCR amplification were examined. Fig. 15D shows the schematic diagram of the oriCdueM9T, oriCdueM9A, oriCdueR13T, and oriCdueR13A sequences created in Example 14-1. These sequences are mutants of which the DUE-M or DUE-R has poly A or poly T sequences. The underline is the same as that in Fig. 15A. As in Fig. 13, each base number was assigned starting from the region 70 bp upstream from the base immediately before the base of the R1 DnaA-box. Using these mutant oriC cassettes, circular DNA amplification was performed in the same manner as in the real-time RCR method in Example 14-2. The doubling time (minutes) of the DNA amplification and standard deviations thus calculated are shown at the rightmost part of Fig. 15D. "N.D." indicates that no DNA amplification was detected.

The five sequences in Fig. 15D are shown in Table 27. In Table 27, the letters enclosed by boxes represent bases different from those of the wild-type sequence, and the shaded letters represent consecutive KAK sequences. The underlined regions represent the M and R elements of the DUE from the 5' side.

**[Table 27]**

| Cassette name | Sequences of DUE (M and R) portions | SEQ ID NO |
|---|---|---|
| oriCwt | AGAGATCTGTTCTATTGTGATCTCTTATTAGGATCG | 104 |
| oriCdueM9T | AGTTTTTTTTTCTATTGTGATCTCTTATTAGGATCG | 105 |
| oriCdueM9A | AGAAAAAAAAACTATTGTGATCTCTTATTAGGATCG | 106 |
| oriCdueR13T | AGAGATCTGTTCTATTGTTTTTTTTTTTTTTGATCG | 107 |
| oriCdueR13A | AGAGATCTGTTCTATTGTAAAAAAAAAAAAAGATCG | 108 |

As shown in Fig. 15D, although the DNA amplification was possible by the RCR method, the doubling time was slower than that of oriCwt (31.9 minutes) in both cases of using the DUE-M mutants, which were sequences with the DUE-M mutated to poly A (oriCdueM9A, 42.8 minutes) and poly T (oriCdueM9T, 35.3 minutes), decreasing by 0.7-fold and 0.9-fold, respectively. Meanwhile, the amplification rate of the DUE-R mutant having poly T (oriCdueR13T, 67.3 minutes) was much lower than that of the DUE-M mutant having poly A or T, and no DNA amplification was observed in the DUE-R mutant having poly A (oriCdueR13A). It was considered that reducing the GC content by adding poly A or T to the DUE-MR region significantly suppresses the RCR amplification without increasing the amplification rate. These results also suggested that the repeated AT motifs in the DUE-M and the KAK motifs in the DUE-R contributed to the enhancement of RCR amplification.

In addition, when the same experiment was conducted using a sequence with increased KAK motifs in the R element (GTATTATTATTAGgat (SEQ ID NO: 115); the uppercase letters represent the DUE-R, the lowercase letters represent the 3' sequence, and the underlines represent mutations from the wild type), the doubling time of the DUE-R mutant was 29.2 ± 2.0 minutes, also indicating that the KAK motifs in the DUE-R contributes to the enhancement of RCR amplification.

When the same experiment was conducted using a sequence with increased AT motifs in the DUE-R (GTATATATATTAGgat (SEQ ID NO:116); the uppercase letters represent the DUE-R, the lowercase letters represent the 3' sequence, and the underlines represent mutations from the wild type), the doubling time of the DUE-R mutant was increased compared to the wild type.

### [Example 14-5]

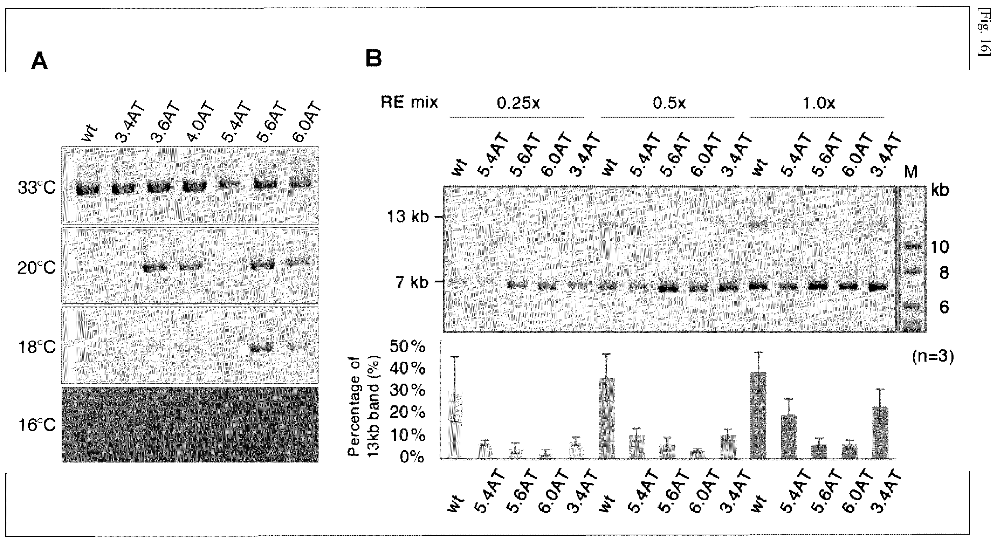

The possibility of RCR amplification at low temperatures with oriC cassette mutants was examined. Amplification was performed by the RCR method on 1 pM 7-kb circular DNAs having oriCwt, oriC3.4AT, oriC3.6AT, oriC4.0AT, oriC5.4AT, oriC5.6AT, and oriC6.0AT, which were created in Example 14-1, at 16°C, 18°C, 20°C, or 33°C for 12 hours. The results of performing agarose gel (1%) electrophoresis (AGE) on the amplification products are shown in Fig. 16A.

As shown in Fig. 16A, when oriC5.6AT and oriC6.0AT were used, high amplification was observed even at 18°C. When oriC3.6AT and oriC4.0AT were used, slight amplification was observed at 18°C. When oriC5.6AT and oriC6.0AT were used, slight amplification was observed even at 16°C. The mutations in the DUE had an effect of promoting the RCR amplification at low temperatures, and the effect was particularly enhanced with the mutation that resulted in consecutive KAK sequences in the DUE-R. It was considered that the consecutive KAK sequences may enhance the cleavage of double-stranded DNA and improve the amplification efficiency at low temperatures compared to oriC3.6AT.

Next, competition tests were conducted using oriCwt, oriC3.4AT, oriC5.4AT, oriC5.6AT, and oriC6.0AT created in Example 14-1. The amplification of 7-kb circular DNA including each oriC was carried out using 13-kb circular DNA including oriCwt as a competitor. The 7-kb DNA and 13-kb DNA were mixed at a ratio of 100 pM:200 pM and amplified using an RCR reaction mixture containing an enzyme mix (RE mix) for amplification in the RCR method at a final concentration of 0.25×, 0.5×, or 1.0×. The results of separating the amplification products by agarose gel (1.0%) electrophoresis are shown in Fig. 16B. The lower graph in Fig. 16B shows the abundance (%) of 13-kb DNAs calculated from the band intensity using the NIH ImageJ software (Schneider, C. A., Rasband, W. S., and Eliceiri, K. W. (2012) NIH Image to ImageJ: 25 years of image analysis. Nature methods, vol. 9(7), p. 671-675.).

As shown in Fig. 16B, oriC3.4AT and oriC5.4AT competed against oriCwt, increasing binding of DnaA to the DnaA box. These results suggested a greater tendency for binding to the DnaA mutant over binding to the wild type. It was considered that these mutants were more competitive with lower levels of DnaA without increasing the amplification rate. It was also found that oriC5.6AT was more likely to be cleaved with low concentrations of DnaA compared to the wild-type sequence (data not shown).

### [Example 15]

The same experiment as in Example 5 was performed except that the plasmid obtained in Example 4 which was the E1-p15A plasmid incorporating OriCsg and the plasmid incorporating OriC5.6ATsg instead of OriCsg were used, and the RCR amplification reaction conditions were adjusted to a temperature of 30°C and reaction time of 6 hours, 10 hours, 15 hours, or 20 hours. For RCR amplification, the reaction mixture with the composition shown in Table 9 was used after being diluted as appropriate. During the reaction, 0.05% Tween 20 (Wako Pure Chemical Industries, Ltd.) was added.

The OriC5.6ATsg cassette has an OriCsg cassette sequence (SEQ ID NO: 10) in which oriC is the same as the oriC of OriC5.6ATsg. The base sequence is shown in Table 28.

**[Table 28]**

| OriC5.6ATsg cassette | SEQ ID NO |
|---|---|
| gagctgttgacagagggtcattttcacactataatgccgctatagtgagtcgtagtatgttgtacctaaagGG ACCTGTTTTTGATGCCATCAGAAAACGCGTCAGCGCCGCTCTGAGGCAATAAACAGAATCAGGCATAAAATCAG CCGCACAGATTTTTTAAAACGCGCCACGGGATTTTTAAACCGGTATTTAACGGTGTATGAATCCCGTTTTATCT TCCTTTGGATCCTGGGTATTAAAAAGAAGATCTATTTATTTATATATATATTATATTATTAT  TATTATTA GGATCGCACTGCCCTGTGGATAACAAGGATCCGGCTTTTAAGATCAACAACCTGGAAAGGATCATTAACTGTGA ATGATCGGTGATCCTGGACCGTATAAGCTGGGATCAGAATGAGGGGTTATACACAGCTGAGGAACTGAAGAGCA GTTGTTCTTTGGATAACTACCGGTTGATCCAAGCTTCCTGACAGAGTTATCCACAGTAGATCGCACGATCTGTc agctcatttcctttagGtacaacatactagaatatttgcctacagcctcctttcggaggctgtttttta | 109 |

In the table, the regions of underlined uppercase letters represent SGS, as in OriCsg. The region near the center indicated by uppercase letters (without underline) represents the same oriC sequence as that in OriC5.6AT. In the table, the portion indicated by bold letters is the DUE portion, and the portions where the bold letters are enclosed by boxes are the mutated portions in the mutant DUE. The underlined portions are the M element (underlined portion on the 5' end side) and the R element (underlined portion on the 3' end side; has a single-base deletion mutation) of the DUE. The regions enclosed by boxes represent the terG16 sequence and the sequence complementary thereto.

The OriC5.6ATsg cassette was incorporated into the p15A plasmid in the same manner as in Example 1. OriC_FW_1 and OriC_RV_1 in Table 8 were used as primers. Then, cloning was performed by the same method as in Example 4-1, and a plasmid in which OriC5.6ATsg was incorporated into the E1-p15A plasmid was obtained.

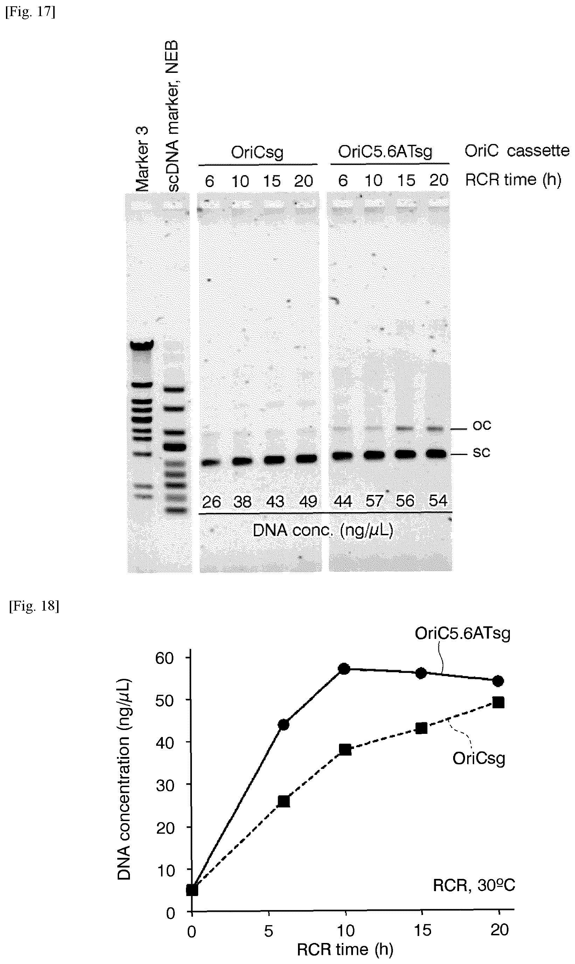

The results are shown in Fig. 17. The change in DNA concentration with reaction time is shown in Fig. 18. When the OriC5.6ATsg cassette having the characteristics of both OriCsg and OriC5.6AT was used, the amplification rate was faster and the concentration of the amplified DNA was higher compared to the case of using the OriCsg cassette.

### [Example 16]

The same experiment as in Example 5 was performed except that the two plasmids obtained by incorporating OriCsg or OriC5.6ATsg into the E1-p15A plasmid were used as in Example 15, and the RCR amplification reaction conditions were adjusted to a temperature of 20°C, 25°C. or 30°C and reaction time of 5 hours, 10 hours, or 20 hours. For RCR amplification, the reaction mixture with the composition shown in Table 9 was used after being diluted as appropriate. During the reaction, 0.05% Tween 20 (Wako Pure Chemical Industries, Ltd.) was added.

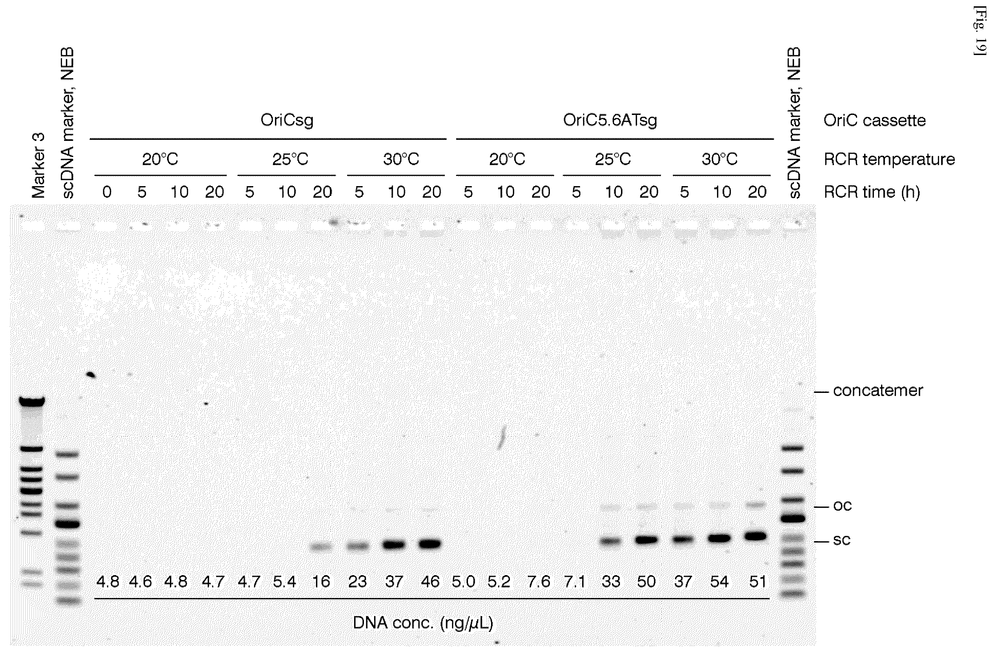

The results are shown in Fig. 19. When the OriC5.6ATsg cassette having the characteristics of both OriCsg and OriC5.6AT was used, DNA amplification was observed in a shorter time even at 25°C, and the DNA was amplified faster even at lower temperatures compared to the case of using the OriCsg cassette.

### [Example 17]

The same experiment as in Example 5 was performed except that the plasmid obtained in Example 4 which was the E1-p15A plasmid incorporating OriC5.6AT and the plasmid obtained in Example 15 which was the E1-p15A plasmid incorporating OriC5.6ATsg were used, and the RCR amplification reaction conditions were adjusted to a temperature of 30°C and reaction time of 6 hours, 10 hours, or 20 hours. For RCR amplification, the reaction mixture with the composition shown in Table 9 was used after being diluted as appropriate. During the reaction, 0.05% Tween 20 (Wako Pure Chemical Industries, Ltd.) was added.

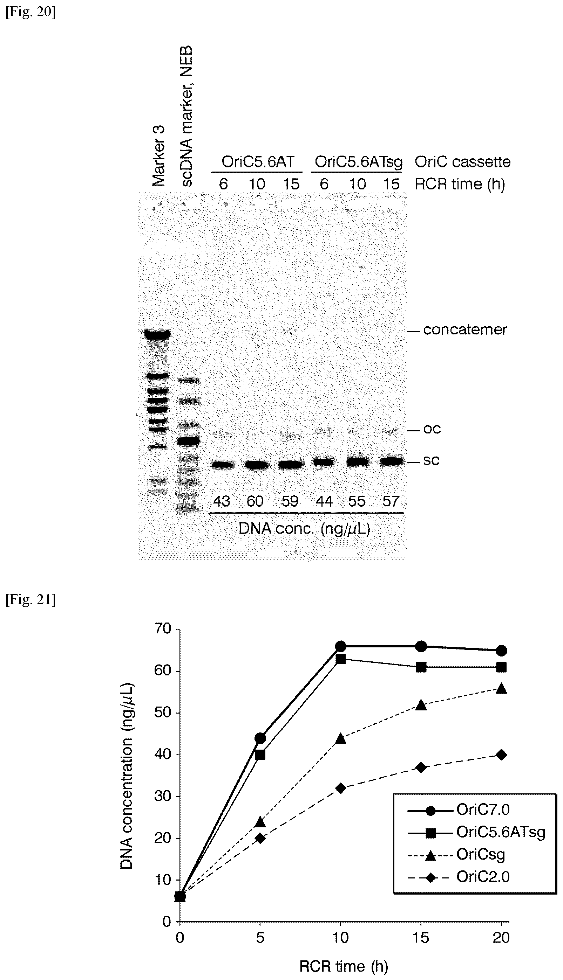

The results are shown in Fig. 20. When an OriC5.6ATsg cassette having the characteristics of both OriCsg and OriC5.6AT was used, fewer concatemers were produced compared to the case of using the OriC5.6AT cassette, and it was considered that more accurate amplification was possible.

### [Example 18]

A cassette was created that had a terG16 sequence and the sequence complementary thereto in proximity to both ends of an oriC sequence having the same mutated DUE portion as that of the OriC5.6ATsg cassette shown in Example 15, and had the same SGS as that of the OriC5.6ATsg cassette further to the 5' side of the oriC sequence and the terG16 sequence, and this cassette was named OriC7.0 cassette.

The following plasmids were prepared.
·A plasmid obtained in Example 15 which was E1-p15A plasmid incorporating OriC5.6ATsg
·A plasmid obtained similarly by incorporating OriC7.0 into the E1-p15A plasmid
·Two types of plasmids obtained in Example 4 which were E1-p15A plasmids incorporating each of two OriC cassettes (OriC2.0 and OriCsg)

The same experiment as in Example 5 was performed except that the RCR amplification reaction conditions were adjusted to a temperature of 30°C and reaction time of 5 hours, 10 hours, 15 hours, or 20 hours. For RCR amplification, the reaction mixture with the composition shown in Table 9 was used after being diluted as appropriate. During the reaction, 0.05% Tween 20 (Wako Pure Chemical Industries, Ltd.) was added.

The changes in DNA concentration with reaction time are shown in Fig. 21. Similar to the results of Example 15, when the OriC5.6ATsg and OriC7.0 cassettes having the characteristics of both OriCsg and OriC5.6AT were used, the amplification rate was faster and the concentration of the amplified DNA was higher compared to the case of using the OriCsg cassette.

### [Example 19]

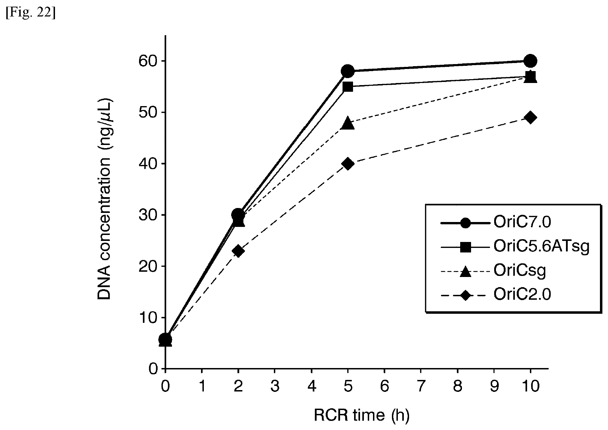

The same experiment as in Example 18 was performed except that the RCR amplification reaction conditions were adjusted to a temperature of 37°C and reaction time of 2 hours, 5 hours, or 10 hours. The changes in DNA concentration with reaction time are shown in Fig. 22. Similar results were obtained to those in Example 18, showing higher DNA amplification rate at a reaction temperature of 37°C than at 30°C (Example 18).

## Claims

1. A method for producing circular DNA, the method comprising:
(1) preparing circular DNA serving as a template, the circular DNA including (i) a replication initiation sequence capable of binding to an enzyme that has a DnaA activity, and (ii) a gyrase binding sequence, and
the circular DNA not including a DnaA box cluster at a different position from the replication initiation sequence;
(2) forming a reaction mixture of the circular DNA prepared in (1) and a reaction liquid including
a first enzyme group that catalyzes replication of the circular DNA,
a second enzyme group that synthesizes two sister circular DNAs forming a catenane, and
a third enzyme group that catalyzes a separation reaction of the two sister circular DNAs; and
(3) incubating the reaction mixture formed in (2) at a temperature of 80°C or lower, thereby obtaining a reaction product that includes the circular DNA, which has been amplified.

2. The method according to claim 1, wherein the replication initiation sequence in (i) has a mutant double-strand cleavage region with a higher AT content than the double-strand cleavage region of a wild-type replication initiation sequence, the double-strand cleavage region having L, M, and R elements.

3. The method according to claim 1, wherein the gyrase binding sequence in (ii) is a sequence derived from bacteriophage Mu.

4. A method for producing circular DNA, the method comprising:
(1) preparing circular DNA serving as a template, the circular DNA including (i) a replication initiation sequence which is capable of binding to an enzyme having a DnaA activity, and
has a mutant double-strand cleavage region with a higher AT content than the double cleavage region of a wild-type replication initiation sequence, the double-strand cleavage region having L, M, and R elements;
(2) forming a reaction mixture of the circular DNA prepared in (1) and a reaction liquid including
a first enzyme group that catalyzes replication of the circular DNA,
a second enzyme group that synthesizes two sister circular DNAs forming a catenane, and
a third enzyme group that catalyzes a separation reaction of the two sister circular DNAs; and
(3) incubating the reaction mixture formed in (2) at a temperature of 80°C or lower, thereby obtaining a reaction product that includes the circular DNA, which has been amplified.

5. The method according to claim 4, following (3), further comprising:
(4) diluting the reaction product two-fold or more with a solution that does not contain the first to third enzyme groups, and maintaining the diluted product obtained after the dilution under conditions in which the second enzyme group and the third enzyme group act.

6. The method according to claim 2 or 4, wherein the mutant double-strand cleavage region is
a mutant double-strand cleavage region in which, among the L, M, and R elements constituting the double-strand cleavage region, the M and/or R elements have a higher AT content than the wild-type M and/or R elements,
a mutant double-strand cleavage region in which the M element constituting the double-strand cleavage region has repetitive AT sequences, and/or
a mutant double-strand cleavage region in which the R element constituting the double-strand cleavage region has four or more consecutive KAK (K is T or G) motif sequences.

7. The method according to claim 1 or 4, wherein the circular DNA further includes a ter sequence inserted outward with respect to the replication initiation sequence,
the ter sequence is not adjacent to the replication initiation sequence, and
the reaction liquid in (2) further includes a protein having an activity of inhibiting replication by binding to the ter sequence.

8. The method according to claim 1 or 4, wherein the circular DNA further includes a ter sequence inserted outward with respect to the replication initiation sequence capable of binding to an enzyme having a DnaA activity,
the ter sequence is a ter sequence with the introduction of a mutation causing a substitution, deletion, or insertion of one or more bases in the wild-type ter sequence, and
the reaction liquid in (2) further includes a protein having an activity of inhibiting replication by binding to the ter sequence.

9. The method according to claim 1 or 4, wherein the incubation in (3) includes isothermal incubation.

10. The method according to claim 1 or 4, wherein the incubation in (3) includes incubation under temperature cycles repeating incubation at two temperatures of 65°C or lower.

11. The method according to claim 1 or 4, wherein the first enzyme group includes an enzyme having a DnaA activity, an SSB, an enzyme having a DnaB-type helicase activity, an enzyme having a DNA helicase loader activity, an enzyme having a DNA primase activity, an enzyme having a DNA clamp activity, and an enzyme or enzyme group having a DNA polymerase III activity,
the second enzyme group includes an enzyme having a DNA polymerase I activity and an enzyme having a DNA ligase activity, and
the third enzyme group includes an enzyme having a topoisomerase III activity or an enzyme having a topoisomerase IV activity.

12. The method according to claim 1 or 4, wherein the reaction liquid further includes a linear DNA-specific exonuclease.

13. The method according to claim 1 or 4, wherein the reaction liquid further includes a single-stranded DNA-specific exonuclease.

14. Circular DNA, comprising:
(i) a replication initiation sequence capable of binding to an enzyme that has a DnaA activity; and (ii) a gyrase binding sequence,
wherein the circular DNA does not include a DnaA box cluster at a different position from the replication initiation sequence.

15. Circular DNA, comprising:
(i) a replication initiation sequence capable of binding to an enzyme that has a DnaA activity; (ii) a gyrase binding sequence; and (iii) a plasmid replication origin,
wherein the circular DNA does not include a DnaA box cluster at a different position from the replication initiation sequence.
